# EUROPEAN PATENT APPLICATION

(11) **EP 4 197 585 A1**
(43) Date of publication of application: **21.06.2023**
(21) Application number: 23156157.2
(22) Date of filing: 21.02.2020
(51) Int. Cl.: A61M 60/10, A61M 60/13, A61M 60/174, A61M 60/237, A61M 60/414, A61M 60/808, A61M 60/81, A61M 60/824, A61M 60/825

(54) **CATHETER DEVICE WITH A DRIVE SHAFT COVER**

(30) Priority: 22.02.2019 EP 19158904
(62) Divisional of application: 20706494.0
(71) Applicant: ECP Entwicklungsgesellschaft mbH, 52074 Aachen (DE)
(72) Inventor: SIESS, Thorsten, 52074 Aachen (DE); SCHECKEL, Mario, 52074 Aachen (DE); SCHUMACHER, Jörg, 52074 Aachen (DE); DECKE, Robert, 52074 Aachen (DE)
(74) Representative: Pfenning, Meinig & Partner mbB

(57) **Abstract**

The invention relates to a catheter device (1), comprising a drive shaft (4) extending from a driving region (16) of the catheter device (1) to a distal end region (8) of the catheter device (1), a rotor (2) which is attached to the drive shaft (4) in the distal end region (8) and a distal bearing (9) for bearing a distal end of the drive shaft (4). The distal bearing comprises a drive shaft cover (11) which is configured to cover a section of the drive shaft (4) extending distally of the rotor (2). On a distal side of the rotor (2), a radially inner part of the rotor (2) is recessed with respect to radially outer parts of the rotor (2) to form a hollow space (2.3) surrounding the drive shaft (4), wherein a proximal end of the drive shaft cover (11) lies in said hollow space (2.3).

## Description

The application relates to a catheter device with a rotor, comprising a drive shaft, according to the preamble of the main claim.

Such catheters are typically used as blood pump arrangements, where the device is positioned in the body of a human or animal, to produce or transmit a torque or rotation movement, such that the rotor effects a flow of blood. The drive shaft runs axially along the longitudinal extension of the catheter between a driving region of the catheter and a distal end region of the catheter. Typically, the driving region is located in a proximal end region, which remains outside the body and is connected to a drive motor. Therefore, the drive shaft should remain pliable and flexible, also under load.

For many applications, it is necessary to guide the catheter along a desired path through the body, for example, along or within blood vessels, in order to position the rotor located at the distal end of the catheter at a desired location within the body, for example, within a heart ventricle of near a heart ventricle, for the duration of the respective application. The rotor and drive shaft then rotate in a rotating direction, according to the desired application, for example, such that a flow of blood from away from the patient's heart, in a proximal direction, is effected. In order to guide the catheter through a lumen, the catheter device can be designed as an expandable pump, where the rotor is designed as a radially compressible rotor, which can be arranged inside a radially compressible housing. Both the rotor and the housing can be transferred into a cannula or sheath, which is typically located proximally of the rotor and has an inner diameter that is smaller than the diameter of the rotor and the housing in an expanded state. For example, by exerting a pulling force on a pliable sheath provided around the drive shaft at the proximal end of the catheter device, the compressible rotor and the compressible housing can be transferred at least in part into the cannula or sheath, and are thereby compressed.

For example, for delivering blood, it can be necessary to produce rotation speeds of more 10,000, more than 20,000 or even more than 30,000 revolutions per minute. Often, the rotation movement must be produced over a longer period of time, such as for several days or even weeks.

For some arrangements, the provision of a distal bearing for stabilizing the distal end of the drive shaft has benefits. In some embodiments, the distal bearing can comprise an elongated polymer part wherein the drive shaft is mounted. The polymer part can for instance be made of Pebax^{®} or Polyurethane or Polyetheretherketon (PEEK). Furthermore, additional bearings, for instance made of ceramics, can be provided inside the elongated polymer part.

Typically, catheter devices of this type comprise a flexible atraumatic tip to avoid damage to the patient's tissue. The atraumatic tip can be made of a flexible medical grade polymer such as Pebax^{®} or Polyurethane. Preferably, the flexible atraumatic tip is designed as a pigtail.

In some embodiments, the elongated polymer end part and the flexible atraumatic polymer tip form a single polymer end part.

Particularly high demands are placed on the mechanical and the chemical loadability of the catheter, the drive shaft, and in particular the distal bearing, which can be in contact with the rotating shaft and can therefore be subject to physical forces leading to heavy abrasive wear. At high rotational speeds, frictional heat is produced, in some cases leading to temperatures of over 160°C, thus exceeding the melting point of some medical-grade polymers used for making the above-mentioned polymer end part. Under these circumstances, a distal bearing made of such a material would be subject to melting.

Material fatigue and damaging processes on the drive shaft and the distal bearing and on other components should only progress as slowly as possible, and moreover as predictably and as controllably as possible, as they not only damage the catheter device, but also present a health hazard to the patient, as wear debris is transferred to the blood and into the patient's body. The risk of tearing and breakage of the drive shaft or the distal bearing or melting of the distal bearing should be minimized. In particular, the bearing should be designed to minimize friction and heat production, which are important factors leading to wear and tear.

Frictional forces and heat production are not only damaging to the pump itself. It should also be taken into account that blood consists of a number of constituents, such as blood cells, which can be damaged both mechanically when in contact with the rotor and the shaft or other parts of the catheter device, or thermally when exposed to the heat produced within the catheter device, for instance due to denaturation. Certain blood proteins will decompose at 60°C so that this can be seen as an upper acceptable limit.

Furthermore, damage to patient tissue caused by the rotating elements should be avoided. For example, intraventricular pumps can cause damage to the heart, as heart tissue, such as for instance tendinous chords or structures pertaining to the mitral valve, can be sucked into the pump or become entangled with rotating parts.

To avoid entanglement of tissue with rotating parts, EP 2047873 describes Polyurethane drive shaft covers which separate the rotating drive shaft from the blood. For this purpose, the gap between the drive shaft and the drive shaft cover is kept very small. However, this can lead to increased wear and tear, especially when flexible drive shafts made out of metal are used. On the other hand, a rigid tube-shaped drive shaft cover requires precise centering of the flexible drive shaft inside the drive shaft cover. EP 2868331 describes a flexible pump, which tolerates bending of the pump head.

However, in a device as described in EP 2868331, in particular in conjunction with a flexible polymer end part at the distal end of the pump head, a rigid drive shaft cover can lead to a kink in the drive shaft upon bending of the catheter device. In particular, a kink can form in the region between the rigid drive shaft cover and the rotor, potentially leading to severe damage of the drive shaft.

In such a configuration, friction leads to relevant heat production between the drive shaft and the bearing, in some cases causing both damage to the blood and melting of the plastic of the pigtail tip. While the heat quantity deposited is not particularly large, it is very concentrated in a small area. The resulting energy density is therefore significant and leads to localized high temperatures, especially if the surrounding material comprises a low thermal heat conductivity such as polymer.

The aim of the application is to address the above-mentioned problems, at least to address one or more of the following points:
- Avoiding damage to the surrounding tissue by the rotating parts of the pump, in particular in the distal end region,
- providing sufficient flexibility to allow for bending of the pump head without producing a kink in the drive shaft,
- providing sufficient resistance to wear and tear and to reduce or avoid transfer of wear debris to the patient's body,
- allowing for a flexible plastic tip at the distal end of the pump, such as a pigtail tip,
- allowing for heat transfer of the generated frictional heat to the surrounding blood to avoid local overheating
- increasing the longevity and durability of the pump,
- avoid clogging of the pump.

This can be achieved by a catheter device according to the independent claim. Advantageous embodiments are given by the dependent claims and the examples provided in the description.

Said catheter device may comprise a drive shaft extending from a driving region of the catheter device, where a motor may be located for driving the drive shaft, to a distal end region of the catheter device. In the distal end region, a rotor may be attached to the drive shaft such that it can rotate together with the drive shaft. A distal bearing can be provided for bearing a distal end of the drive shaft. The distal bearing may comprise a drive shaft cover which is configured to cover a section of the drive shaft extending distally of the rotor. This way, during operation of the catheter device, it can be avoided that for instance tissue, such as tendinous tissue or trabeculae or muscle bridges, might get caught in said section of the drive shaft which is covered by the drive shaft cover.

The distal bearing may comprise an end part which may be designed to be brought in contact with for example tissue of a patient when the catheter pump is used in the patient. The drive shaft cover may be provided in a section of the drive shaft lying between the rotor and the end part, in particular along the entire section. In an embodiment of the catheter device, a distal end of the drive shaft cover may be provided in the end part, i.e. the drive shaft cover may extend into the end part.

On a distal side of the rotor, a radially inner part of the rotor may be recessed with respect to radially outer parts of the rotor to form a hollow space surrounding the drive shaft. In this case, the recessed radially inner part extends less in a distal direction than the outer parts. The hollow space is then open to the distal side.

A proximal end of the drive shaft cover may lie in said hollow space. This means that the end of the drive shaft cover (at which end the drive shaft protrudes from the drive shaft cover and would otherwise be exposed) may be surrounded by the rotor. In this setup, an opening at the proximal end of the drive shaft cover where the drive shaft protrudes, may be in fluid communication with a volume defined by the hollow space.

Having the rotor around the end of the drive shaft cover in the above-described fashion thus helps to protect the section of the drive shaft which lies between the end of the drive shaft cover and the hub of the rotor. With this setup, even very small pieces of tissue can be kept from getting caught in the drive shaft or from getting sucked into the distal bearing. At the same time, the end of the drive shaft cover may remain at a safe distance from the hub of the rotor to avoid that the rotor might contact the drive shaft cover during operation. In a possible design, there is no portion of the rotor hub extending distally from the rotating rotor blades.

The hollow space typically has a cylindrical shape. It may be designed concentrically with the drive shaft and a hub of the rotor. The radially inner part which is recessed may for instance comprise a distal section of the hub or it may be comprised by the distal section of the hub. In one embodiment, the hollow space is provided in the hub of the rotor.

The drive shaft cover may be designed as a hollow tube. It can be essentially cylindrical. Thereby, an inner diameter can be chosen in accordance with a diameter of the drive shaft. The inner diameter and/or the outer diameter of the drive shaft cover may vary along a length of the drive shaft cover. In particular, having two or three cylindrical sections with different outer diameters can be particularly advantageous in view of the desired functionality of the drive shaft cover, as will be explained here below.

The hub of the rotor may be designed in such a way, that touching of parts can be avoided. For example, the hub of the rotor may be designed to extend less than 0.5 mm past the rotor blades in the distal direction, in order to be able to bring the rotor blades closer to the distal bearing or the end part without the hub potentially touching parts of the drive shaft cover. Preferably, the hub extends less than 0.1 mm in distal direction past the rotor blades, particularly preferably the hub does not extend at all past the rotor blades on the distal side, i.e., the hub can be flush with a leading edge of the rotor blades.

Inside the hollow space, a radial gap is formed between the drive shaft cover and the rotor, i.e., between an outer surface of the drive shaft cover and a cylindrical surface of the rotor delimiting the hollow space. Furthermore, an axial gap is formed between the proximal end of the drive shaft cover and a hub of the rotor. Both gaps should be kept large enough to avoid touching of the parts while keeping the hollow space as small as possible to avoid taking too much material away from the rotor. Both gaps can furthermore be used to circulate blood through the gaps by a pumping function of the drive shaft to avoid any blood stagnation or clotting or local overheating.

A length of the hollow space, which is measured in axial direction, i.e. along the drive shaft, may be for instance at least 0.5 mm, preferably at least 0.7 mm, particularly preferably at least 0.9 mm. Additionally or alternatively, the length may be chosen to be at most 2.5 mm, preferably at most 1.5 mm, particularly preferably at most 1.1 mm.

The drive shaft cover may in one embodiment extend at least 0.3 mm, preferably at least 0.4 mm into the hollow space. On the other hand, it can for instance be chosen to extend at most 2.2 mm, preferably at most 0.8 mm, particularly preferably at most 0.7 mm into the hollow space. This length may be called penetration depth.

The catheter device may be designed as an expandable pump, wherein the rotor is located in a housing. The housing and the rotor can then be configured to be compressed at least along a radial direction extending transversely to a longitudinal direction, from an expanded state into a compressed state. Upon compression of the housing, a relative motion of the rotor with respect to the distal bearing can be effected. This can be due to a change in length of the housing which is associated with the compression of the housing. Said change in length thus gives rise to the aforementioned relative motion.

The penetration depth of the drive shaft cover can be chosen such that the proximal end of the drive shaft cover remains within the hollow space in the compressed state. I.e. the penetration depth can in particular be larger than the above-described change in length of the housing.

The axial gap is preferably provided in such a way, that under a typical deformation of the pump that is to be expected during use of the pump, the proximal end does not come into contact with any parts of the rotor, i.e., the axial gap should allow axial displacement which occurs due to elastic deformation of the pump housing during use. In particular, it can be provided in an embodiment of the catheter device, that a change in length of the axial gap that is due to an apical impulse can be tolerated in such a way, that a gap of at least 0.1 mm remains. In general, the axial gap can thus be adjusted, depending on the elastic properties of the pump.

In an exemplary embodiment, a gap size of the axial gap between the proximal end of the drive shaft cover and the hub of the rotor may be for instance at least 0.2 mm, preferably at least 0.3 mm and/or at most 1.5 mm, preferably at most 0.9 mm, particularly preferably at most 0.6 mm, to avoid touching of the parts.

The above-mentioned radial gap is also designed in such a way, that touching of the drive shaft cover and the rotor is avoided. At the same time, the hollow space should be kept as small as possible, so that potential weakening of the hub is minimized.

In an exemplary embodiment, a gap size of the radial gap between the drive shaft cover and the rotor may be chosen to be at least 0.01 mm, preferably at least, 0.04 mm, particularly preferably at least 0.07 mm and/or at most 0.2 mm, preferably at most 0.13 mm. It was found that ovalization of the rotor - in particular of the rotor hub and thus of the hollow space - during operation can be tolerated without touching of the parts when a gap as described here is provided.

In order to be able to make the hollow space as small as possible while maintaining a desired radial gap size, a wall thickness of a portion of the drive shaft cover penetrating the hollow space may for instance be at least 0.03 mm, preferably at least 0.05 mm, and/or at most 0.3 mm, preferably at most 0.08 mm, particularly preferably at most 0.07 mm.

Diameters of for instance the drive shaft cover or the gap are chosen in accordance with a diameter of the drive shaft, i.e., gaps or wall thicknesses as mentioned above can be maintained for all typical diameters of the drive shaft. An outer diameter of the drive shaft can for instance lie between 0.4 mm and 2 mm, preferably lies between 0.6 mm and 1.2 mm, particularly preferably between 0.8 mm and 1.0 mm.

In an embodiment of the catheter device, a diameter of the hollow space (measured orthogonally to the axis of the drive shaft, i.e. in radial direction) may be at least 0.5 mm, preferably at least 0.8 mm, particularly preferably at least 1.1 mm. Additionally or alternatively, the diameter of the hollow space may be chosen to be at most 2 mm, preferably at most 1.5 mm, particularly preferably at most 1.3 mm.

It can be advantageous to provide a section with an increased outer diameter distally of a proximal section penetrating the hollow space. The proximal section may then have the above-described wall thickness of the portion penetrating the hollow space. The proximal section of the drive shaft cover extends into the hollow space, with a portion of the proximal section typically remaining outside of the hollow space. In this case, the outer diameter of the drive shaft cover increases at a distance from the rotor. The section lying distally of the proximal section typically has a wall thickness that is increased with respect to the wall thickness of the proximal section. Thereby, an inner diameter may be constant along the two sections or it may change. In particular, the inner diameter may be increased in the section lying distally of the proximal section.

The outer diameter of a proximal section of the drive shaft cover which penetrates into the hollow space can for instance be at least 0.1 mm smaller than the outer diameter of the section lying distally thereof (with increased outer diameter), preferably at least 0.14 mm smaller. Additionally or alternatively, it may be at most 0.6 mm smaller than the outer diameter of the section lying distally of the proximal section, preferably at most 0.3 mm smaller.

In an embodiment where a section with increased outer diameter of the above-described type is envisioned, the wall thickness of the portion of the drive shaft cover penetrating the hollow space may preferably be chosen to be less than 0.3 mm, such as for instance at most 0.08 mm or at most 0.07 mm. It is also possible not to provide an increased diameter of this type. In such an embodiment, the wall thickness of the portion of the drive shaft cover penetrating the hollow space may be the same wall thickness as in a further section of the drive shaft cover lying between the rotor and the end part. In such an embodiment the wall thickness may be for instance up to the above-mentioned 0.3 mm.

The proximal section with reduced outer diameter may have a length of at least 0.6 mm, preferably at least 0.8 mm, particularly preferably at least 0.9 mm. Additionally or alternatively, it may have a length of for instance at most 2 mm, preferably at most 1.5 mm, particularly preferably at most 1.1 mm.

The difference in outer diameter between the proximal section of the drive shaft cover and the section lying distally thereof (being for instance between 0.14 mm and 0.3 mm as mentioned above) may be chosen to be for example twice the radial gap size (meaning the difference in radius is at least equal to the radial gap size). This way, upon axial alignment of the rotor and the drive shaft cover, the section with increased diameter lying distally of the proximal section is larger in diameter than the hollow space. This helps to prevent tissue from entering the hollow space.

A portion of the proximal section remaining outside of the hollow space may be provided to avoid touching of the drive shaft cover and the rotor. Similar to the case of the axial gap, upon typical bending of the catheter device, changes in length are expected and touching of the parts should be avoided by providing a sufficient distance between the distal end of the rotor and the section of increased radius. The portion of the proximal section remaining outside of the hollow space may for instance be chosen to have the same length as the axial gap. It may for instance have a length of at least 0.2 mm, preferably at least 0.3 mm and/or at most 1.5 mm, preferably at most 0.9 mm, particularly preferably at most 0.6 mm i.e., in this case, at a distance of at least 0.2 mm, preferably at least 0.3 mm and/or at most 1.5 mm, preferably at most 0.9 mm, particularly preferably at most 0.6 mm from the distal end of the rotor, the outer diameter of the drive shaft cover may increase. The increase in outer diameter occurs for instance smoothly over an axial section of for instance 0.2 mm or less, preferably 0.1 mm or less.

In a possible embodiment of the catheter device, the rotor may comprise a stiffening element. The stiffening element may be designed to surround the hollow space and can help to prevent or reduce deformation of the rotor, in particular of the rotor hub, during operation. It may for instance be designed as a tube, hollow cylinder or ring-structure that is connected to a material of the rotor.

The stiffening element may be made of a material of a higher stiffness than a material of the rotor, i.e., of the hub and/or the blades of the rotor.

The stiffening element may be moulded into the material of the rotor such that it is completely surrounded by the material of the rotor, in particular it may be moulded into the hub of the rotor. This means that in this case there is additional material of the rotor provided on the inside of the stiffening element. It is however also possible to provide the stiffening element around the hollow space such that the stiffening element itself delimits the hollow space, i.e., with the inner surface of the stiffening element being exposed and an outer surface of the stiffening element being connected to the material of the rotor.

The stiffening element may be provided along the full length of the hollow space. The stiffening element may thereby extend past the hollow space in proximal direction. For example, the stiffening element may be longer than the hollow space, e.g. 1.5 times as long or twice as long as the hollow space.

The stiffening element may comprise structures to provide better attachment to the rotor. For example, microstructures and/or macrostructures can be provided. The microstructures and/or macrostructures can for instance be designed as protrusions, indentations or holes. The microstructures and/or macrostructures can be provided on the outside and/or on the inside of the stiffening element. The microstructures and/or macrostructures may be provided over a whole length of the stiffening element or over part of the length of the stiffening element.

In a possible embodiment, the stiffening element comprises structures of the above-described type, which are designed as one or more anchoring elements extending radially on the outside of the stiffening element. The one or more anchoring elements may be designed and positioned in such a way that they extend past the hub of the rotor and into a material of the rotor blades. In this case, they are preferably designed to allow compression of the rotor for insertion of the rotor into the heart. This can be achieved by having the anchoring elements penetrate into the material of the blades only to an extent where the blades can still be compressed of folded. The anchoring elements may further comprise one or more recesses, indentations or undercuts into which the material of the rotor may penetrate. To allow compression or folding of the blades, in a possible embodiment of the catheter pump, anchoring elements extend for example 0.5 mm into the material of the blades, such that the blades remain compressible.

As mentioned, the stiffening element may, additionally or alternatively, comprise holes or indentations to provide a better connection with the material of the rotor. In particular in the case where the stiffening element is completely surrounded by the material of the rotor, one or more holes which may be designed as through-holes or blind holes may be provided in the tube, allowing the material of the rotor to penetrate through a wall of the stiffening element and enabling a particularly reliable connection between the rotor and its stiffening element. A cross section of the holes or indentations is typically not limited to a specific geometry. They may be circular or polygonal. The indentations or holes may for instance have a diameter or edge length of at least 0.02 mm preferably 0.03 mm and/or at most 0.5 mm, preferably at most 0.1 mm.

The stiffening element may be made of a bio-compatible material. This should in particular be the case in embodiments where the inner surface of the stiffening element is exposed. The stiffening element may for instance comprise one or more of MP35N, 35NLT, Nitinol, stainless steel (in particular medical grade stainless steel), and ceramics.

A wall thickness of the stiffening element may be for instance at least 0.03 mm, preferably at least 0.04 mm and/or at most 0.08 mm, preferably at most 0.07 mm.

It can be advantageous to provide the drive shaft cover with a pliable section to enable bending of the drive shaft. In this case, wear and tear on the drive shaft should be reduced and it should be ensured that no kink occurs in the drive shaft. The embodiments described here below are advantageous when it comes to safely bearing the drive shaft while allowing bending of the drive shaft as it is required when introducing the pump or during operation. In particular, bending of the drive shaft while it is rotating at full operating speed is possible. Having the pliable section in conjunction with other aspects of the application can be particularly advantageous.

The pliable section can be provided between a distal end of the rotor and a proximal end of the end part. In particular, the pliable section may be the section lying distally of the proximal section with decreased diameter or it may be a portion of that section.

The pliable section can for instance be provided by having at least one opening in the drive shaft cover in the pliable section. The at least one opening is then typically designed as a through-opening, connecting an inside of the drive shaft cover to an outside of the drive shaft cover.

The openings in the pliable section may be provided such that due to an elasticity of the material of the drive shaft cover, a restoring force brings the drive shaft cover back into an original straight position after bending.

The at least one opening in the pliable section may comprise one or more slits. The slit or slits may have a course with a tangential component. In particular, one or more slits with a spiral shape can be provided, such that the pliable section forms a spiral sleeve.

If one or more slits with a spiral course are provided, a pitch of the spiral course may be for instance at least 0.2 mm, preferably at least 0.3 mm, particularly preferably at least 0.5 mm and/or at most 1.2 mm, preferably at most 0.9 mm, particularly preferably at most 0.8 mm.

The one or more openings may be cut into the drive shaft cover using a laser. Edges of the one or more openings may be smoothened or rounded to avoid wear and/or tissue damage.

Slits may for instance have a width of at least 0.005 mm, preferably at least 0.01 mm, particularly preferably at least 0.025 mm and/or at most 0.2 mm, preferably at most 0.1 mm.

In one embodiment, the pliable section features several slits following a single course, for instance a single spiral course, the several slits being separated by bridges of material between ends of the slits.

If one or more slits are provided, holes may be provided at one end or at both ends of one or more of the slits, the holes having a diameter that is larger than a width of a given slit at which they are provided. This can improve the durability of the drive shaft cover as it can help to prevent tear propagation along a course defined by the slit.

The pliable section of the drive shaft cover may also be designed as a so-called hypotube, i.e., the slits may be cut in such a fashion, that a particular hypotube-design is provided in the pliable section of the drive shaft cover.

In one embodiment, the slits may have a closed course, completely surrounding the drive shaft cover and thereby cutting the drive shaft cover into several segments having no material bridges between them. The segments can be connected to each other for instance by having protrusions of one segment lying inside recesses of a neighboring segment, resembling pieces of a jigsaw-puzzle. It is also possible to have disconnected segments that are only held together by the flexible tube.

In the case of disconnected segments or in some designs, in particular some known hypotube-designs, the pliable section may be limp, i.e. the drive shaft cover itself will not restore its original shape after deformation. In this case, the flexible tube may have a "memory" characteristic and help restore the original shape of the drive shaft cover.

The slits of the pliable section may be designed to limit bending of the pliable section, i.e. allow bending only up to a given minimal bending radius.

Thus bending can be ensured and in some cases also be limited by the width and the course of the slits. Thereby the minimal bending radius can be limited to a bending radius which does not permanently deform or kink the drive shaft.

Having one or more slits or holes can have the effect that blood may penetrate through the slits or holes. It can be a desired feature to enable a flow of blood, for instance from the inside of the drive shaft cover to the outside.

In some embodiments, a flexible tube may be provided around the pliable section of the drive shaft cover. The flexible tube can for instance be a shrink hose. The flexible tube can be used for tuning the bending properties of the pliable section, for instance stiffening the pliable section to a desired degree.

The flexible tube may comprise a polymer or be made of a polymer. In particular it may comprise or be made of silicone and/or Pebax^{®} and/or PU and/or PET.

If one or more openings such as holes or slits are provided, the one or more openings may be covered by the flexible tube at least in part.

It is possible to leave a section or a subset of the one or more openings uncovered, to locally enable the above-described flow of blood. In particular, the flexible tube may be designed to leave a distal portion of the at least one opening uncovered. Thereby, blood can enter between the drive shaft and the drive shaft cover from within the hollow space of the rotor and can be delivered up to the most distal opening within the drive shaft cover. Thus blood can circulate along the drive shaft to prevent overheating and blood stasis.

Additionally or alternatively, the flexible tube may comprise one or more holes to allow fluid communication with a portion of the at least one opening to enable the above-described flow of blood.

Additionally or alternatively, fluid communication enabling a flow of blood as described above may be provided by having one or more venting holes in the drive shaft cover, the venting holes connecting the inside of the drive shaft cover and the outside of the drive shaft cover. The venting holes may for instance be provided in a region where the flexible tube is not provided, in particular distally from the flexible tube. The venting holes may have a design that is different from the design of the openings, In particular, the venting holes, as opposed to the openings, do not have to render the drive shaft cover pliable. Therefore, the venting holes may have a design that can be optimized for the envisioned through-flow of blood. Furthermore, in the case of the venting holes, the design can be such that clogging and suction of tissue into the venting holes can be avoided. In other words, if venting holes are provided, all of the openings of the pliable section may be covered and a blood flow can still be ensured. This way, both the venting holes and the openings can both be optimized with regard to their primary purpose. The venting holes may for instance have a circular or elliptical shape or they may be designed as slits having a course with an axial component or only an axial component.

In one embodiment, a distal end of the drive shaft cover lies within the end part, with a portion of the drive shaft cover extending into the end part. The drive shaft cover may comprise a distal section with a diameter that is larger than a diameter of a section of the drive shaft cover lying proximally thereof. Said distal section may be designed to lie entirely or in part inside the end part.

The section lying proximally of the distal section may be the section lying distally of the proximal section, amounting to a total of three sections with different outer diameters. I.e., in a possible embodiment of the catheter device, there is a proximal section (extending into the hollow space) with a first outer diameter, a pliable section lying distally thereof, with a second (increased) outer diameter, and a distal section extending into the end part, with a third (further increased) outer diameter. The outer diameter of the distal section may be at least 1.15 mm, preferably at least 1.25 mm and/or at most 2 mm, preferably at most 1.8 mm, particularly preferably at most 1.6 mm.

An inner diameter of the drive shaft cover may also vary over the length of the drive shaft cover. For instance, at the proximal end of the drive shaft cover, the diameter may be reduced with respect to an inner diameter of the drive shaft cover at the distal end of the drive shaft cover. The reduction in diameter may for instance occur between the proximal section and the section lying distally thereof. The inner diameter may be reduced by at least 0.02 mm and/or by at most 0.12 mm. The inner diameter can for instance be kept constant along the pliable section and the distal section. A gap between the drive shaft and the drive shaft cover may be kept particularly small in the proximal section to keep the rotor and the drive shaft cover concentrically aligned. In particular, a difference in diameter between the inner diameter of the drive shaft cover in the proximal section and the outer diameter of the drive shaft may be chosen to be 0.1 mm or less, preferably 0.06 mm or less, particularly preferably 0.03 mm or less.

The drive shaft cover may comprise MP35N, 35NLT and/or ceramics and/or a diamond-like-carbon coating.

The drive shaft cover may be manufactured from a single piece. In particular it may be designed as a single piece. It is however also possible, that slits are cut into the drive shaft cover such that several segments are formed. These segments may be held together but not connected to each other in the sense that no material bridges of the material of the drive shaft cover exist (see above). These slits can be cut into an originally provided single piece. The heat conductivity can depend on the course of the slits. In particular, advantageous heat conducting properties can be provided by having more material bridges.

The drive shaft cover may have heat conducting properties enabling a heat transfer away from the end part.

The end part may comprise an atraumatic tip. The end part can for instance be made of a polymer. It can have an elongated portion, wherein the atraumatic tip is provided distally thereof. The atraumatic tip can be attached to the elongated portion, in particular the elongated portion and the atraumatic tip can be designed as a single piece. The atraumatic tip can for instance be a pigtail.

The drive shaft is typically flexible. The drive shaft can be made up of a plurality of coaxial windings, preferably with different winding directions, particularly preferably with alternating winding directions, running spirally around a cavity extending axially along the drive shaft. For example, a drive shaft can comprise two coaxial windings, with opposite winding directions, and an outer diameter of the drive shaft can lie between 0.4 mm and 2 mm, preferably lies between 0.6 mm and 1.2 mm, particularly preferably between 0.8 mm and 1.0 mm.

In the distal end region, the drive shaft is in some embodiments reinforced by a reinforcement element, for example a metal wire or a carbon wire, that is provided in the cavity extending axially along the drive shaft. In one embodiment, the reinforcement element extends from an area near the proximal end of the rotor housing, in particular from a proximal bearing configuration of the rotor housing to the distal end of the drive shaft. In one embodiment the metal wire is made of 1.4310 stainless steel.

The flexible tube can for instance be made of a flexible material such as silicone, Pebax^{®}, PU or PET. In one embodiment, the flexible tube of the drive shaft cover is a shrink hose. The flexible tube of the drive shaft cover can also be provided on the outside of the end part, extending beyond the end part proximally of the end part. Alternatively or additionally, a flexible tube of the drive shaft cover is provided in part inside the end part, extending beyond the end part proximally of the end part. As the drive shaft bends during operation, the drive shaft cover is sufficiently flexible to avoid a kink in the drive shaft between the drive shaft cover and the rotor. Its elasticity can also allow the drive shaft cover to bend. The bending stiffness is typically mostly defined by the drive shaft cover and the drive shaft.

In another alternative embodiment, the flexible tube can be provided around the drive shaft cover, proximally of the end part and in a manner distanced from the end part. In this case, one or more openings of the drive shaft cover can be provided in a section of the drive shaft cover which lies between the end part and the flexible tube. I.e., these openings are not covered by the flexible tube. In this case, a stream of blood between the inside of the drive shaft cover and the outside of the drive shaft cover can be enabled through the uncovered openings. In particular, the openings can be the aforementioned openings of the drive shaft cover which are at the same time configured to ensure flexibility of the drive shaft cover. This configuration can also be useful in embodiments that do not feature the rotor with the hollow space as described above. It is also possible to provide holes or openings in the flexible tube to allow for a stream of blood between the inside of the drive shaft cover and the outside of the drive shaft cover, while providing the flexible tube for instance along the whole length of the drive shaft cover or along the whole length of the portion having openings.

In one embodiment, the drive shaft cover comprises a spiral sleeve on the inside of the flexible tube for bearing the drive shaft. The spiral sleeve supports the flexible tube of the drive shaft cover from the inside, while ensuring flexibility. With such a spiral sleeve, friction between the drive shaft and the drive shaft cover, as well as wear and tear on the drive shaft cover, can be reduced.

In another embodiment, the drive shaft cover comprises a heat conducting part, or several heat conducting parts, designed to conduct heat away from the drive shaft and/or conduct heat away from the distal bearing. For instance, the heat conducting part can be configured to transfer heat to the blood of the patient during operation and/or to distribute the heat to avoid local hotspots.

The heat conducting part or the heat conducting parts may have an inner side, facing the drive shaft, and an outer side, facing away from the drive shaft.

The heat conducting part may be designed as a tube surrounding the drive shaft. The heat conducting part can for example also be designed as one or more metal plates or tongues which are provided near the drive shaft.

The spiral sleeve and the heat conducting part or tube can each be provided in separate embodiments, for instance in conjunction with a flexible tube. An embodiment featuring both a spiral sleeve and a heat conducting part designed as a tube can be particularly advantageous.

The spiral sleeve can for example be provided in conjunction with a heat conducting part, both with or without the flexible tube. For instance, the spiral sleeve can be arranged at least in part inside the heat conducting part designed as a tube, typically extending out of the tube.

The spiral sleeve and the heat conducting part can also be designed as a single piece. The spiral sleeve can be the pliable section of the drive shaft cover.

The spiral sleeve can for instance be made of round wire or it can be made of flat tape with a winding. The drive shaft is then also rotatably mounted within the spiral sleeve. The bearing spiral sleeve is preferably made of metal, for instance made of MP35N^{®} or 35NLT^{®}, or made of ceramics. The bearing spiral sleeve ensures the flexibility of the drive shaft cover to tolerate bending of the pump head, thus avoiding a kink between the distal bearing and the rotor, and providing sufficient resistance to wear and tear. Thus bending is ensured and in some cases also limited by the defined gap between adjacent windings of the spiral sleeve. Thereby the minimal bending radius can be limited to a bend radius which does not permanently deform or kink the drive shaft. In one embodiment, the flexible tube is provided around the full length of the spiral sleeve. In one embodiment, the flexible tube is provided only around a proximal portion of the spiral sleeve. In one embodiment, the flexible tube is provided around the outside of a portion of the end part and around a portion of the spiral bearing extending out of the end part.

Alternatively, an embodiment of multiple metal rings instead of a spiral is possible, preferably arranged with gaps between the rings. Preferably the rings or the sleeve are made of flat tape. The rings can be made of the same material as the spiral sleeve described above.

A spiral sleeve or rings for bearing a drive shaft have an inner diameter ranging between 0.4 mm and 2.1 mm, preferably between 0.6 mm and 1.3 mm, particularly preferably between 0.8 mm and 1.1 mm. The tape forming the spiral sleeve or rings has a thickness between 0.05 mm and 0.4 mm. The tape forming the spiral sleeve or the rings can for instance have a width between 0.4 and 0.8 mm. The gap between the rings or between the windings can for instance be between 0.04 mm and 0.2 mm.

The winding slope of the spiral sleeve and the thickness of the flexible tube, which influence the flexibility of the drive shaft cover, are preferably chosen such that the rotor can be kept at the desired position upon bending of the catheter device.

The thickness of the flexible tube can be between 5 µm and 100 µm, preferably between 10 µm and 50 µm.

In one embodiment, the inner diameter of the spiral sleeve or rings is chosen to be between 0.01 mm and 0.08 mm larger than the outer diameter of the drive shaft, preferably between 0.01 mm and 0.05 mm, for mounting the drive shaft rotatably and avoiding vibrations, while allowing at most small amounts of blood to enter the gap region.

In one embodiment, the proximal end of the spiral sleeve or rings, is located close to the rotor in the expanded state. For instance, the proximal end of the spiral sleeve or rings can be designed to have a distance of between 0.2 mm and 0.7 mm from the rotor in the expanded state, preferably a distance between 0.25 mm and 0.4 mm, to avoid that the rotor touches the drive shaft cover or spiral sleeve during operation.

Preferably, the flexibility of the drive shaft cover is such that upon bending of the pump head, the drive shaft and the rotor remain centered within the flexible housing, to avoid that the rotor touches the flexible housing during operation.

In one embodiment, a hub of the rotor extends less than 0.5 mm past the rotor blades in the distal direction, in order to be able to bring the rotor blades closer to the distal bearing without the hub potentially touching parts of the distal bearing. Preferably, it extends less than 0.1 mm in distal direction past the rotor blades, particularly preferably the hub does not extend at all past the rotor blades on the distal side.

In one embodiment, the winding direction of the spiral sleeve, when following the winding of the sleeve in the distal direction, when looking from the proximal end to the distal end of the bearing sleeve, is the opposite direction of a preferred rotating direction of the drive shaft, when looking along the drive shaft towards the distal end of the drive shaft, such that a tapered or pointed end of the spiral sleeve would not damage a rotor rotating in the preferred rotating direction if the rotor touches the spiral sleeve in the event of failure. The preferred winding direction can be the same direction as the winding direction of the outermost coaxial winding of the drive shaft or it can be the opposite direction from the winding direction of the outermost coaxial winding of the drive shaft.

The ends of the spiral sleeve are preferably face ground and the edges, at least the edges of both ends, are rounded and smooth, preferably with a ten-point mean roughness R_{z} of R_{z}≤2µm, according to the ISO 1302 standard.

In another embodiment, a proximal section of the drive shaft cover is provided proximally of the spiral sleeve, the proximal section extending into the hollow space of the rotor. This means that the ends of the spiral sleeve are protected by the proximal section of the drive shaft cover.

Preferably, the spiral sleeve and/or the drive shaft cover is arranged in such a manner, that, if a force is exerted at the proximal end of the catheter device to transfer the rotor and the housing into a cannula under compression, such that a relative motion of the drive shaft with respect to the distal bearing and therefore the spiral sleeve or the drive shaft cover is effected. The relative motion is for instance due to a change in length of the housing which is effected by compression of the housing, as described above. The distal end of the drive shaft can remain within the distal bearing at all times, i.e., depending on the embodiment, the distal end does not escape the drive shaft cover, the spiral sleeve, the ceramic bearing or the heat conducting tube.

In one embodiment, an additional ceramic bearing is provided within the distal bearing, located distally of the spiral sleeve.

As mentioned earlier, the catheter device can comprise a heat conducting part or tube in addition to the spiral bearing or the catheter device can comprise a heat conducting part or tube solely in combination with a bearing.

If the heat conducting part or tube is provided without the spiral bearing, a ceramic bearing, for example a ring bearing, can be provided inside the distal bearing.

If the heat conducting part or tube is provided in addition to the spiral sleeve, it can be provided around at least a portion of the spiral sleeve.

The heat conducting part or tube can lie in part within the end part and in part outside of the end part. Thus, heat transfer from within the distal bearing to the blood of the patient is enabled. In one embodiment, the heat conducting part or tube extends between 0.5 mm and 2 mm out of the end part, preferably between 1 mm and 1.5 mm.

The flexible tube of the drive shaft cover can be provided around the spiral bearing on the inside of the heat conducting part or tube. Then, an outer side of the heat conducting part or tube can be brought in direct contact with the blood of the patient.

The flexible tube can also be provided around the outside of a portion of the end part, the outside of a portion of the heat conducting part or tube extending out of the end part, and a portion of the spiral sleeve that extends beyond the heat conducting part or tube. In the latter configuration, the part of the heat conducting part or tube, which extends out of the end part, cannot be brought in direct contact with the blood. Rather, the flexible tube is in direct contact with the blood. In this configuration, heat is also transferred from the heat conducting part or tube to the blood, through the thin walls of the flexible tube.

The heat conducting part or tube can also lie entirely within the end part, such that the heat is redistributed within the distal bearing and conducted away from the spiral bearing or the rings.

The heat conducting part or tube is for instance made of a medical grade stainless steel, such as 1.4441 stainless steel, and possesses a higher thermal conductivity than the end part or the ceramic bearing.

An inner diameter of the heat conducting part designed as a tube can lie between 0.5 mm and 2.6 mm, preferably between 0.7 mm and 1.8 mm, particularly preferably between 0.9 mm and 1.6 mm.

The thickness of the heat conducting part or tube can be between 0.05 mm and 0.5 mm.

The section of the outer surface of the heat conducting part or tube which is configured to be in contact with the blood of a patient is preferably smooth.

In one embodiment, the ten point mean roughness R_{z} according to the ISO 1302 standard in said section or portion of the outer surface of the heat conducting part is R_{z}≤1.2 µm.

In one embodiment, the inner side of the heat conducting part or tube is configured to be glued to the spiral sleeve. To facilitate gluing the inner side of the heat conducting part or tube to the spiral sleeve, the inner side of the part or tube can be rough. For instance, the arithmetic average surface roughness of the inner side of the heat conducting part or tube can have an average surface roughness Rₐ according to the ISO 1302 standard of Rₐ≥0.8 µm.

In one embodiment, the inner diameter of the heat conducting part designed as a tube is chosen to be between 0.04 mm and 0.1 mm larger than the outer diameter of the spiral sleeve or the rings so that glue can be applied in the gap.

Such catheter pumps with a heat conducting part or tube can result in shifting of the temperature hot-spot. For example, the hot spot can be shifted from a region of the drive shaft that lies inside the end part to a closer to the proximal end of the end part, or to a region which lies outside of the end part. Such a setup can also result in a lower maximum temperature, for example a maximum temperature which is between 20°C and 60°C lower than the maximum temperature in a setup without heat conducting part. In particular, the maximum temperature at the hotspot can be kept below the melting point of Pebax^{®} or other medical grade polymers.

It is also possible to provide a catheter device which features a heat conducting part or tube as presented here, but where the distal bearing does not feature a spiral sleeve or rings.

The present application may further relate to a drive shaft cover as described above or below, and/or to a bearing system comprising the drive shaft cover and the flexible tube.

Aspects and embodiments of the catheter device according to the application are exemplified in Figures 1 to 20.
Figure 1 shows a catheter device which is positioned within the left ventricle of a heart;
Figure 2 shows the distal end region of a catheter device;
Figure 3 shows an enlarged section of the distal end region of a catheter device;
Figures 4a and b show schematic sketches of a section of the distal end region of a catheter device;
Figures 5 a and b show schematic sketches of a section of the distal end region of a catheter device;
Figure 6 shows the spiral sleeve;
Figures 7 a and b show the rotor and the rotor housing in the expanded state (a) and in the compressed state (b);
Figures 8 a and b show the catheter device with a rotor having a hollow space and the drive shaft cover extending into the hollow space;
Figures 9a - c show the catheter device from Figure 8 with an additional flexible tube;
Figures 10 a and b show the catheter device from Figure 8 with a stiffening element provided in the rotor;
Figure 11 shows a detailed view of the catheter device;
Figure 12 shows a detailed view of a catheter device having a rotor with a stiffening element;
Figures 13 a-c show different views of a drive shaft cover in a first embodiment;
Figures 14 a-b show different views of a drive shaft cover in a second embodiment;
Figures 15 a-b show different views of a drive shaft cover in a third embodiment;
Figure 16 a shows a drive shaft cover in a fourth embodiment;

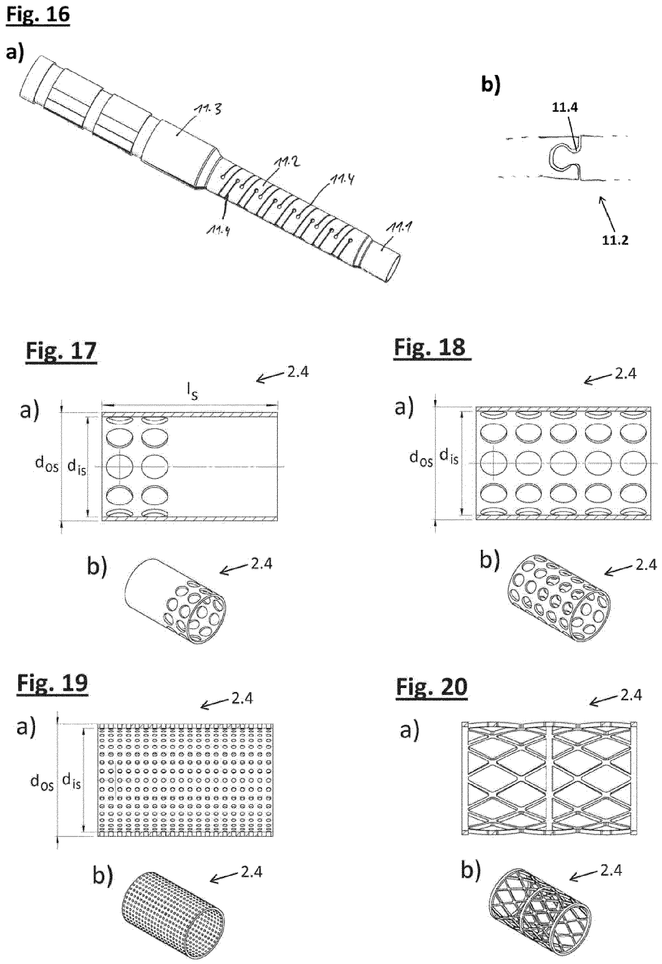

Figure 16 b shows a section of a drive shaft cover in a fifth embodiment;
Figures 17 a-b, 18 a-b, 19 a-b and 20 a-b show different embodiments of the stiffening element, in each case in two different views.

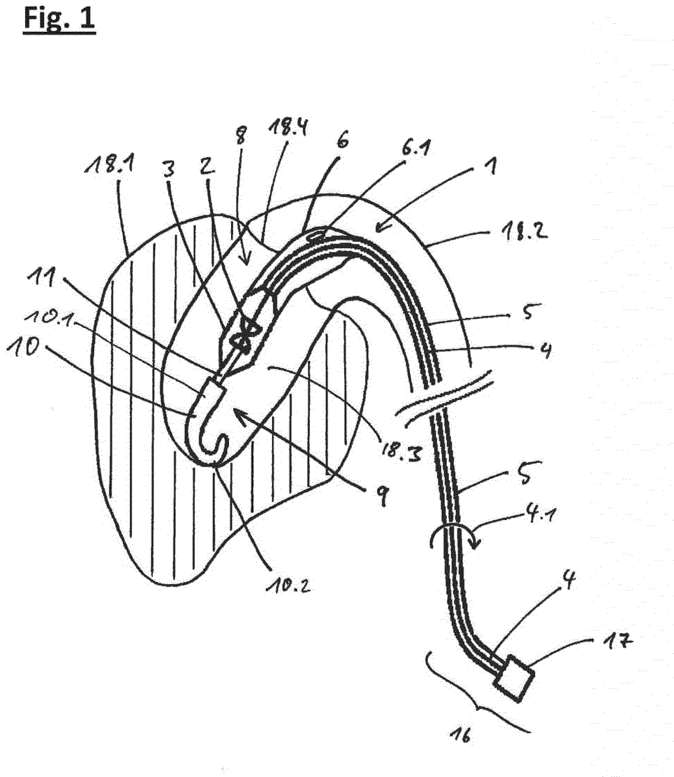

Figure 1 shows a catheter device 1 used as a blood pump. The catheter device 1 is introduced into a patient, such that a portion of the distal end region 8 of the catheter device 1 is positioned within the left ventricle 18.3 of the heart 18.1 of the patient. In a driving region 16 which can lie outside of the patient's body, a motor 17 is provided for driving a drive shaft 4. A portion of the drive shaft 4 is covered by a pliable sheath 5. The drive shaft 4 and the pliable sheath 5 extend from the driving region 16 to the distal end region 8, where a rotor 2, preferably configured as a compressible rotor, is driven by the drive shaft 4. The compressible rotor 2 is located within a compressible housing 3. The compressibility of the rotor 2 and the housing 3 is useful for introducing the rotor into the patient's body at a lower profile. During operation, the rotor 2 and the housing 3 are in an expanded state. The housing 3 prevents damage to heart tissue such as for instance the tendinous chords, as it prevents tissue from being sucked into the rotor 2 or becoming entangled with the rotor 2 or the drive shaft 4. The distal end of the drive shaft 4 lies within a distal bearing 9. The distal bearing comprises a drive shaft cover 11 and a polymer end part 10, the polymer end part preferably made of a flexible material, such as Pebax^{®} or another flexible medical grade polymer, preferably with a "memory" characteristic, i.e. such that it regains its original shape after being deformed. The polymer end part comprises an elongated portion 10.1 which is provided around a part of the drive shaft cover 10. The polymer end part 10 further comprises a pigtail tip 10.2 to prevent damage to the heart 18.1 and the aortic valve 18.4 during pump placement. The rotor 2 and the drive shaft 4 can rotate in a rotating direction 4.1, such that a flow of blood away from the distal end, towards the proximal end is effected, i.e. a blood flow out of the left ventricle 18.3 into the aorta 18.2 and to other regions of the patient's body. A downstream tubing 6 is provided proximally of the rotor 2 and the rotor housing 3, which downstream tubing has a downstream opening 6.1 that lies proximally of the aortic valve 18.4, such that the blood passes the aortic valve within the downstream tubing 6 and can then stream into the aorta 18.2. The downstream tubing 6 is made of a flexible material, such that it can be compressed by the aortic valve 18.4 as the patient's heart 18.1 continues to pump. The downstream tubing 6 is typically expanded mainly due to the active blood flow generated by the rotor 2 during rotation.

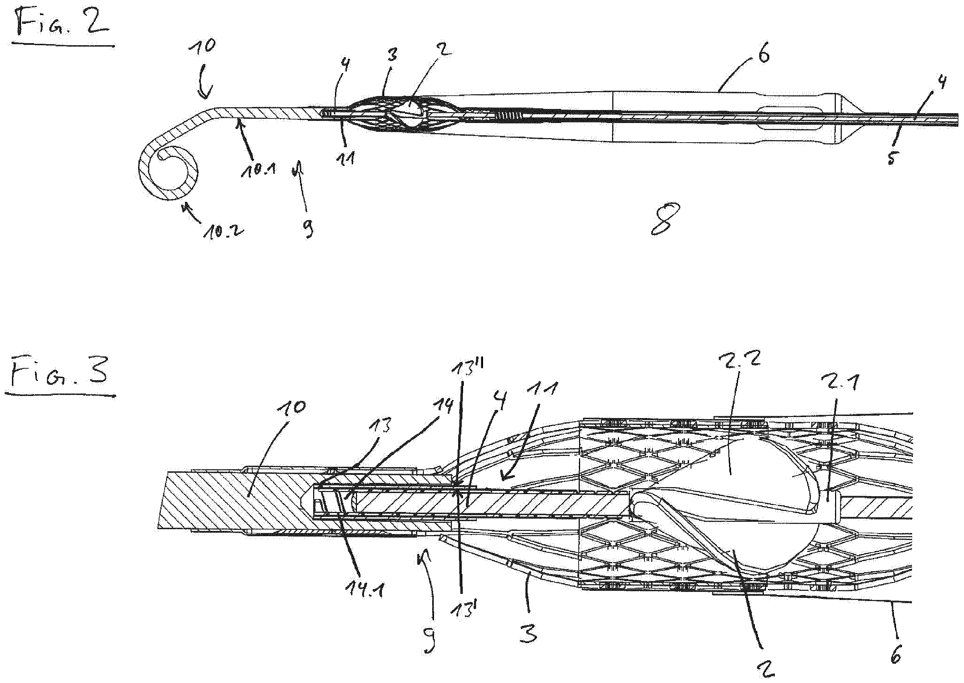

Figure 2 shows a cut through the distal end region 8 of the catheter device 1. The distal bearing 9 comprises the polymer end part 10 with the pigtail 10.2 and the elongated portion 10.1. On the proximal end, the elongated portion 10.1 is provided around a portion of a drive shaft cover 11. The drive shaft 4 extends into the distal bearing 4 and is borne by the drive shaft cover 11. The downstream tubing 6 is attached to the rotor housing 3 and extends proximally. The proximal end of the downstream tubing 6 is attached to the pliable sheath 5. Between the rotor 2 and the proximal side of the drive shaft cover 11, the drive shaft should be protected to avoid damage to the heart. This is achieved by the catheter device described in more detail in the following figures

Figure 3 shows an enlarged portion of the end region 8 of the catheter device 1. In particular, the section of the distal bearing 9 which comprises the drive shaft cover 11 is shown. The drive shaft cover 11 extends from within the polymer end part 10, out of the polymer end part 10, into the rotor housing 3. The drive shaft 4 is made of one or more layers of coaxial windings which run spirally around a cavity extending axially at the center of the drive shaft. The winding direction of the coaxial windings can alternate from one layer to the next. This setup can improve the flexibility of the drive shaft. The outer diameter of the drive shaft lies in a range of about 0.4 to about 2 mm. Preferably, the outer diameter lies between 0.6 mm and 1.2 mm. Particularly preferably, the diameter lies between 0.8 mm and 1.0 mm. The drive shaft cover 11 is designed for bearing the drive shaft 4. It comprises a sleeve with a lumen in which the drive shaft 4 is inserted. The sleeve is preferably designed as a spiral sleeve 14 out of flat tape 14.1. The tape can for instance be made of MP35N^{®} or 35NLT^{®} or ceramics. The inner diameter of the spiral sleeve 14 is chosen such that the drive shaft 4 can be mounted but remains rotatable, while no large amounts of blood can enter the gap between the drive shaft 4 and the spiral sleeve 14. The inner diameter of the spiral sleeve 14 can for instance be chosen to be between 0.01 mm and 0.08 mm larger than the outer diameter of the drive shaft 4, preferably between 0.01 mm and 0.05 mm larger than the outer diameter of the drive shaft 4. The inner diameter of the spiral sleeve 14 is between 0.4 mm and 2.1 mm, preferably between 0.6 mm and 1.3 mm, particularly preferably between 0.8 mm and 1.1 mm. The thickness of the spiral sleeve 14 is between 0.05 mm and 0.4 mm. Such a spiral sleeve 14 provides flexibility, particularly in the region extending out of the polymer end part 10. Preferably, the flexibility of the drive shaft cover 11 is such that a kink in the drive shaft is avoided if the distal end region 8 of the catheter device 1 is bent. Furthermore, the flexibility of the drive shaft cover 11 is such that the drive shaft 4 remains centered within the housing 3 and the rotor 2 does not touch the housing 3. The proximal end of the spiral sleeve, preferably both ends of the spiral sleeve are face ground. Furthermore, the edges of the both ends of the spiral sleeve are rounded and smooth, preferably with a ten-point mean roughness of R_{z}≤2µm, according to the ISO 1302 standard. The drive shaft cover 11 can further comprise a heat conducting part 13 which can be designed as a tube which is provided around a portion of the spiral sleeve 14. The heat conducting tube or part 13 is made of a material with a higher thermal conductivity than the polymer end part 10, in particular it can be made of medical grade stainless steel, such as 1.4441 stainless steel. The heat conducting part 13, when designed as a tube, is provided at least around a portion of the spiral sleeve 14 which lies inside the polymer end part 10, in some embodiments, the heat conducting part 13 or tube extends out of the polymer end part 10, into a region within the housing 3 which can be configured to be in direct contact with the blood of the patient. In particular, the heat conducting part 13 designed as a tube can extend between 0.5 mm and 2 mm out of the polymer end part 10, preferably between 1 mm and 1.5 mm. The heat conducting part 13 or tube can have a thickness of between 0.05 mm and 0.5 mm. An inner diameter of the heat conducting tube can be between 0.5 mm and 2.6 mm, preferably between 0.7 mm and 1.8 mm, particularly preferably between 0.9 mm and 1.6 mm. If the heat conducting part 13 or tube is configured such that a portion of the outer side 13" of the heat conducting part 13 or tube can be brought in direct contact with the blood of the patient, the area of the outer side (13") of the heat conducting part 13 or tube which can be brought in contact with the blood of the patient is preferably smooth, for instance with a ten-point mean roughness of R_{z}≤1.2µm according to the ISO 1302 standard. The portion of the outer side 13" of the heat conducting part 13 which is configured to lie within the polymer end part and be in contact with the polymer end part is preferably roughened, for instance by laser texturing or knurling, preferably with an average surface roughness of Rₐ≥0,8 µm, according to the ISO 1302 standard. On the proximal side of the drive shaft cover 11, the rotor 2 with a rotor hub 2.1 is provided around the drive shaft 4. When in the operating state, in which the rotor is expanded, the rotor hub 2.1 is kept at an axial distance of between 0.2 mm and 0.7 mm from the drive shaft cover, preferably at a distance of between 0.25 mm and 0.4 mm. The hub 2.1 of the rotor is designed such that the rotor blades 2.2 can be brought close to the drive shaft cover 11. The hub 2.1 extends less than 0.5 mm past the rotor blades in distal direction, preferably, it extends less than 0.1 mm or not at all past the rotor blades in distal direction.

The heat conducting part (13), which can be designed as a tube, can be provided inside the polymer end part 10 independently from the spiral sleeve 14, for example if a different kind of bearing or no additional sleeve for bearing the drive shaft 4 is envisioned.

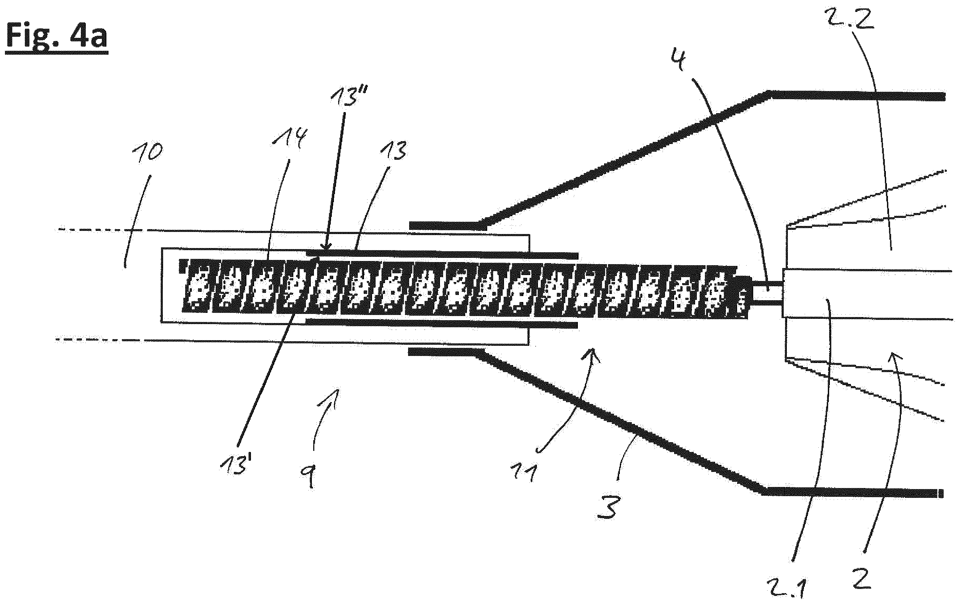

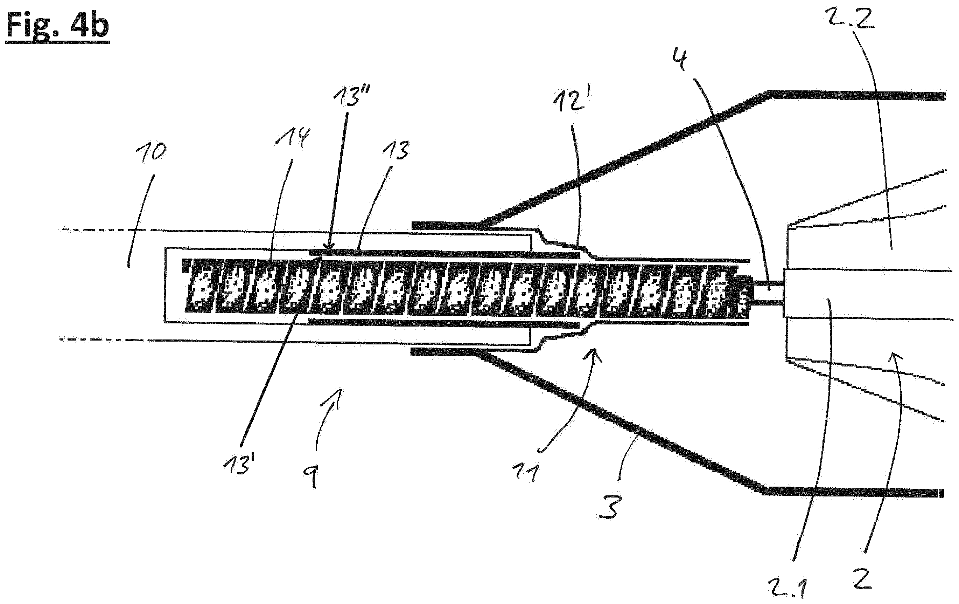

Figure 4 a shows a schematic of a section of the distal end region 8 of the catheter device 1. A portion of the spiral sleeve 14 extends out of the polymer end part 10.The inner side 13' of the heat conducting part is in direct contact with the spiral sleeve 14 and can be rough in order to facilitate gluing the spiral sleeve 14 to the inner side 13' of the heat conducting part 13. The bare portion of the spiral sleeve 14 extending out of the polymer end part 10 is highly flexible and follows even strong bending motion of the drive shaft 4 during operation. A portion of the heat conducting tube 13 also extends out of the polymer end part 10 to enable heat transfer. In this embodiment, heat is transferred from the heat conducting 13 tube directly to the blood. The heat conducting tube 13 can also extend further into the distal bearing 10 and cover the spiral sleeve 14 at least in all areas that lie inside the polymer end part 10. In an alternative embodiment, there is no heat conducting tube 13, but all other features are the same.

Figure 4 b shows a schematic of the same section of the distal end region 8 of the catheter device 1 as Figure 4 a. The drive shaft cover 11 further comprises a flexible tube 12' around the outside of the spiral sleeve or a portion of the outside of the spiral sleeve. In the embodiment shown in Figure 4 b, the flexible tube 12' runs around a proximal portion of the polymer end part 10, around a portion of the outer side 13" of the heat conducting part 13 which reaches out of the polymer end part 10, and around the portion of the spiral sleeve 14 extending out of the polymer end part 10.The inner side 13' of the heat conducting part is in direct contact with the spiral sleeve 14 and can be rough in order to facilitate gluing the spiral sleeve to the inner side 13' of the heat conducting part 13. The flexible tube can be implemented as a shrink hose and can be made for instance of silicone or of Pebax^{®} or of PU or of PET. For good heat conductivity, the flexible tube can have a small wall thickness, for instance smaller than 0.2 mm, in particular smaller than 0.02 mm. In this embodiment, heat is transferred from the heat conducting 13 tube to the blood through the flexible tube 12'. In an embodiment featuring a flexible tube 12', rings made of flat tape can be provided on the inside of the flexible tube 12' instead of a spiral sleeve. They can for example be made of MP35N^{®} or 35NLT^{®} or ceramics and have the same thickness and inner diameter as the spiral sleeve. In a possible embodiment with rings, the rings are arranged distant from each other.

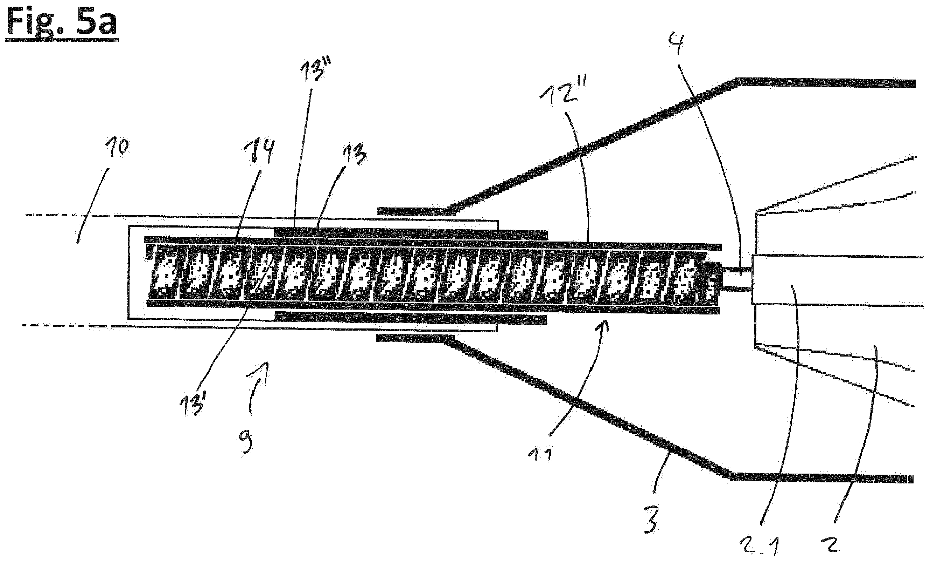

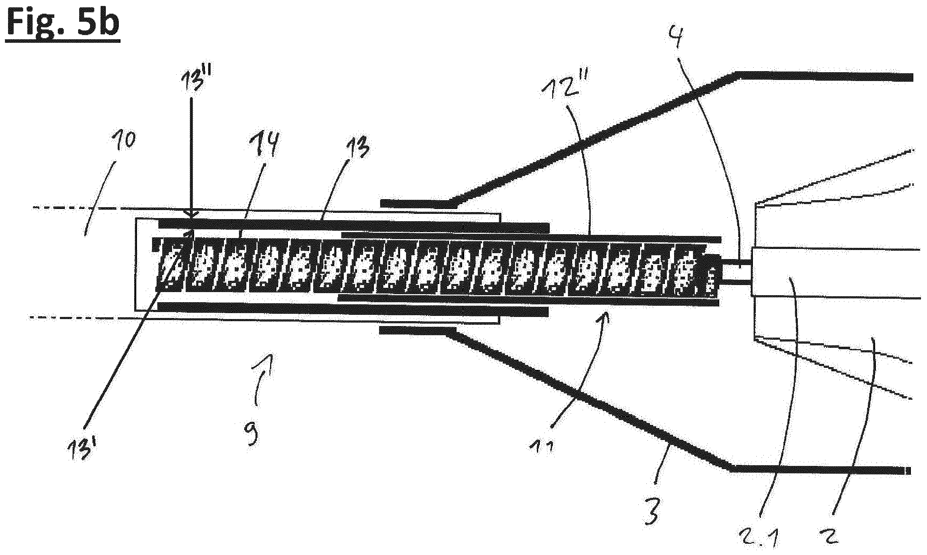

Figure 5 a shows the same section as Figure 4 b, but with a flexible tube 12" in a different configuration. The flexible tube 12" can also be implemented as a shrink hose and be made of for instance of silicone or of PEBAX^{®}, PU or PET. For good heat conductivity, the flexible tube can have a small wall thickness, for instance smaller than 0.2 mm, in particular smaller than 0.02 mm. The flexible tube 12" is provided on the outside of the spiral sleeve 14, and it runs along the inner side 13' of the heat conducting part 13 or tube and inside the polymer end part 10. In the embodiment shown here, the flexible tube 12" extends all the way to the distal end of the spiral sleeve 14. In this configuration, a portion of the outer side 13" of the heat conducting part 13 is configured to be in direct contact with the blood of the patient upon insertion of the catheter device 1 into a patient. Said portion is smooth, for instance with a ten-point mean roughness R_{z}, according to the ISO 1302 standard, of R_{z}≤1.2µm.

Figure 5 b shows a similar configuration as Figure 5 a, with the flexible tube 12" provided on the outside of the spiral sleeve 14, running on inner side (13') of the heat conducting part 13 and inside the polymer end part 10. Different from Figure 5 a, the flexible tube 12" does not extend all the way to the distal end of the spiral sleeve 14 such that a distal portion of the spiral sleeve is not covered by the flexible tube 12". The heat conducting part 13, on the other hand, extends further to the distal end of the spiral sleeve 14 and thus a portion of its inner side 13' is configured to be in direct contact with the spiral sleeve 14. In this configuration, said portion of the inner side 13' of the heat conducting part 13 can be glued to the outside of the spiral sleeve 14. It is advantageous to provide a roughened surface on the inner side 13' of the heat conducting part 13'. For instance, with an average surface roughness of Rₐ≥0.8 µm, according to the ISO 1302 standard. Furthermore, to enable the application of glue between the heat conducting part 13 and the spiral sleeve 14, the heat conducting 13 when designed as a tube can have an inner diameter which is between 0.04 mm and 0.1 mm larger than the outer diameter of the spiral sleeve 14.

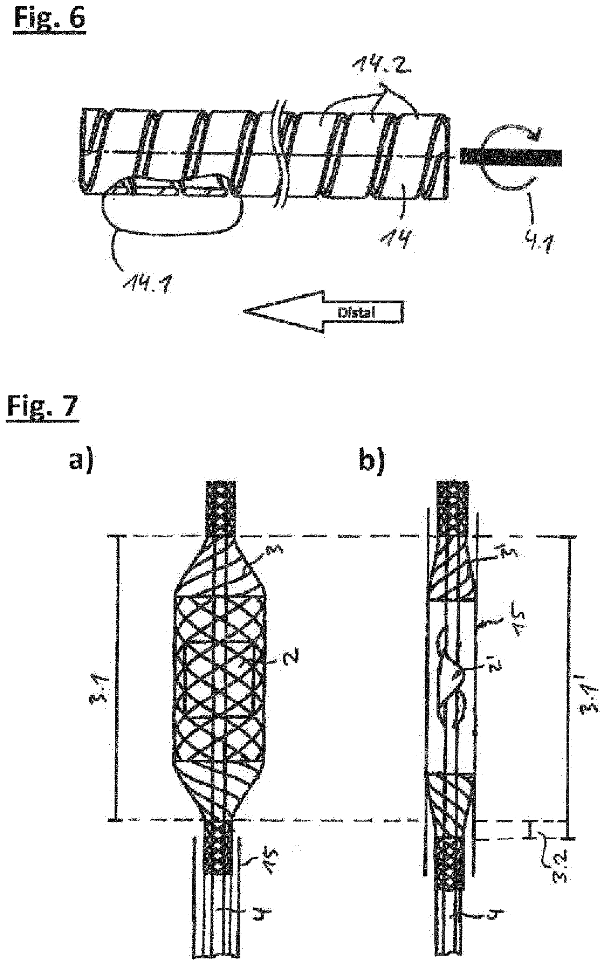

Figure 6 shows the spiral sleeve 14. The ends are face ground and smooth. The flat tape 14.1 is shown in a cut-away. The winding 14.2 has a winding direction from proximal to distal, which is the opposite direction of the preferred rotating direction 4.1 of the drive shaft 4, when looking in distal direction. This way, a rotating part cannot get damaged or caught by a pointed tip at the proximal end of the spiral sleeve 14.

Figure 7 shows the rotor 2 and the housing 3 and a cannula 15 in two states a and b. The rotor 2 and the housing 3 are configured to be transferred into the cannula 15, for instance by exerting a force at the proximal end of the pliable sheath 5. When transferred into the cannula, the rotor 2' and the housing 3' are compressed in a radial direction, from their expanded states 2,3 into their compressed states 2',3'. The cannula 15 can be a cannula pertaining to the catheter device 1 or peel-away-sheath to aid the insertion of the catheter device 1 into the body of a patient. The housing 3 in the expanded state has a length 3.1. As the housing 3 is compressed to the compressed state 3', the length increases to a length 3.1'. As the length changes, the relative position of the distal bearing 9 which is attached to the housing 3 with respect to the drive shaft 4 changes. The drive shaft cover 11 is designed such that the distal end of the drive shaft 4 remains within the drive shaft cover 11 as the housing 3 undergoes changes in length 3.2 while the two parts slide against each other.

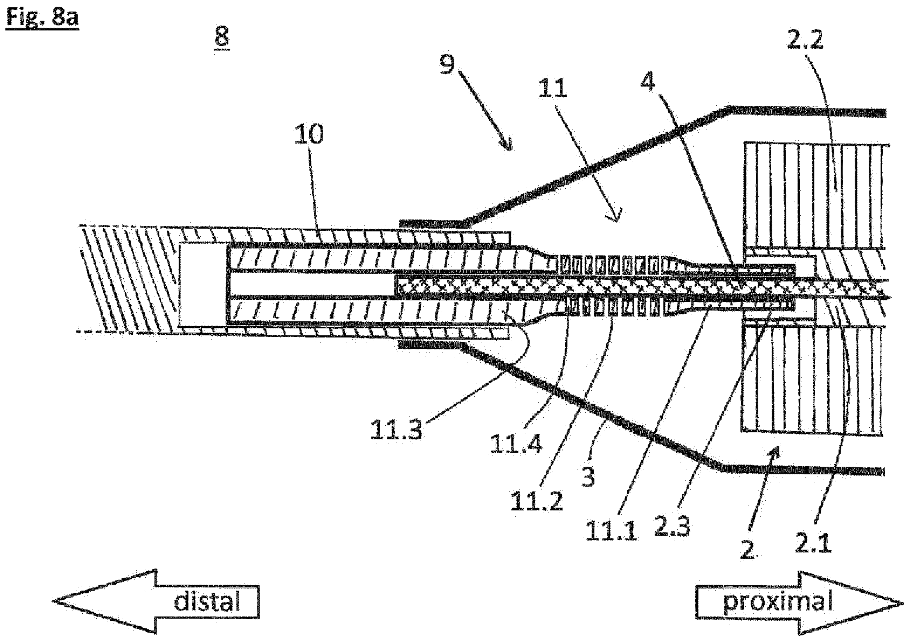

Figure 8a shows a view of the distal end region 8 of the catheter device 1, the catheter device 1 being designed essentially as shown for instance in Figure 1.

The distal bearing 9 is provided for bearing the distal end of the drive shaft 4. The distal bearing 9 comprises the end part 10 and the drive shaft cover 11. The drive shaft cover 11 covers a section of the drive shaft 4 which extends between the rotor 2 and the end part 10. The drive shaft cover 11 thereby covers said section of the drive shaft 4 along a whole length of the section.

On a distal side of the rotor 2, a radially inner part of the rotor 2, in particular of the rotor hub 2.1 is axially recessed with respect to radially outer parts of the rotor 2 and the rotor hub 2.1 to form a hollow space 2.3 surrounding the drive shaft 4. The hollow space 2.3 is cylindrical and open towards the distal side. A proximal end of the drive shaft cover 11 lies in the hollow space 2.3. The section of the drive shaft 4 which protrudes from the drive shaft cover 11 at its proximal end is therefore protected by the portions of the rotor 2 surrounding it.

A proximal section 11.1 of the drive shaft cover 11 partially lies within the hollow space 2.3. The proximal section 11.1 has a first outer diameter. Distally thereof, a second central section 11.2 of the drive shaft cover 11 is provided with a second diameter that is larger than the first diameter. The central section 11.2 comprises one or more openings 11.4 to make it pliable. A distal section 11.3 is provided distally of the central pliable section 11.2. The distal section 11.3 has a third diameter which is larger than the second diameter and extends into the end part 10. A portion of the distal section 11.3 thereby remains outside of the end part 10 to enable efficient heat transfer away from the end part. Heat conductivity is enhanced in this version, since the drive shaft cover 11 is designed as a single heat conducting piece.

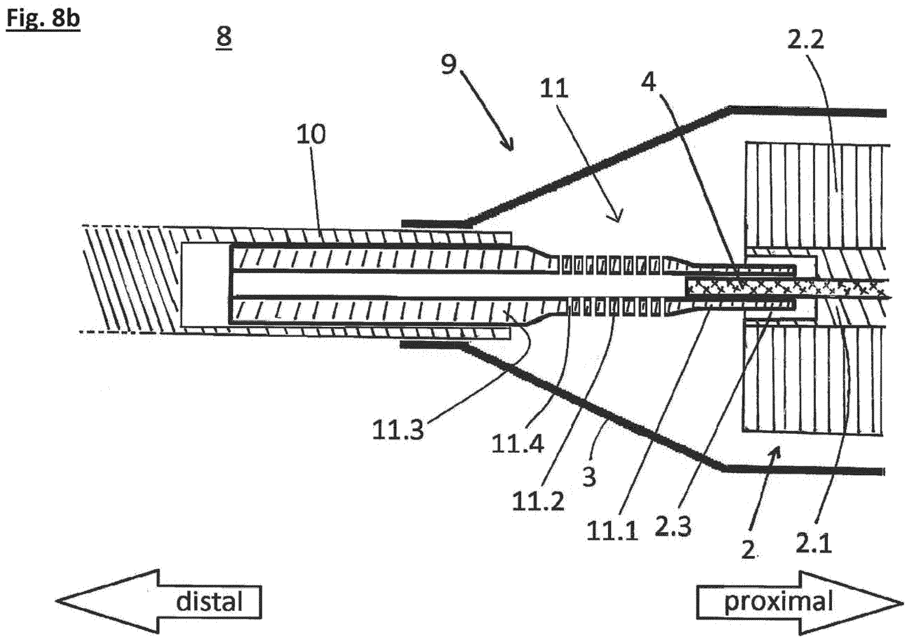

The housing 3, the drive shaft 4 and the drive shaft cover 11 as shown in Figure 8 are designed to enable bending of the catheter device 1, in particular in the section lying between the distal end of the rotor 2 and the proximal end of the end part 10. Safe bending is possible even during operation of the catheter device, as kinks in the drive shaft can be advantageously avoided.

The drive shaft cover 11 comprises metal, for example 35NLT^{®} and/or MP35N^{®}, and/or ceramics and/or a diamond-like-carbon coating. It is manufactured from a single piece and designed as a single piece.

It is also possible to have a drive shaft cover with a different design extend into the hollow space 2.3 of the rotor 2. For instance it is possible, to have the hollow space of the rotor in combination with one of the drive shaft covers shown in Figures 4a to 5b, with the spiral sleeve extending into the hollow space. In this case, the proximal end of the spiral sleeve may be modified to a closed tube-structure, for example by welding, to avoid sharp edges which might damage the rotor, for instance if the spiral sleeves touches the rotor under extreme or unforeseen conditions.

Figure 8b shows a setup that is similar to that of Figure 8a, however the section of the drive shaft 4 extending distally of the rotor 2 is kept shorter than in the case of Figure 8a. Thereby, the drive shaft 4 ends proximally of the pliable section 11.2 of the drive shaft cover 11. The drive shaft 4 extends over a length into the drive shaft cover that is at least the expected change in length 3.2 which the housing 3 undergoes under compression (cf. Figs. 7a, b). This way, the drive shaft 4 does not escape the drive shaft cover in the compressed state, when the drive shaft cover is moved away from the rotor in distal direction. With this setup, damage to the drive shaft 4 in the shown section due to heavy deformation of the drive shaft cover can typically be avoided completely. The flexibility of the pliable central section 11.2 can then be tuned through the design of the openings 11.4 (in conjunction with the material properties of the drive shaft cover 11), without having to take into account the bending properties of the drive shaft 4.

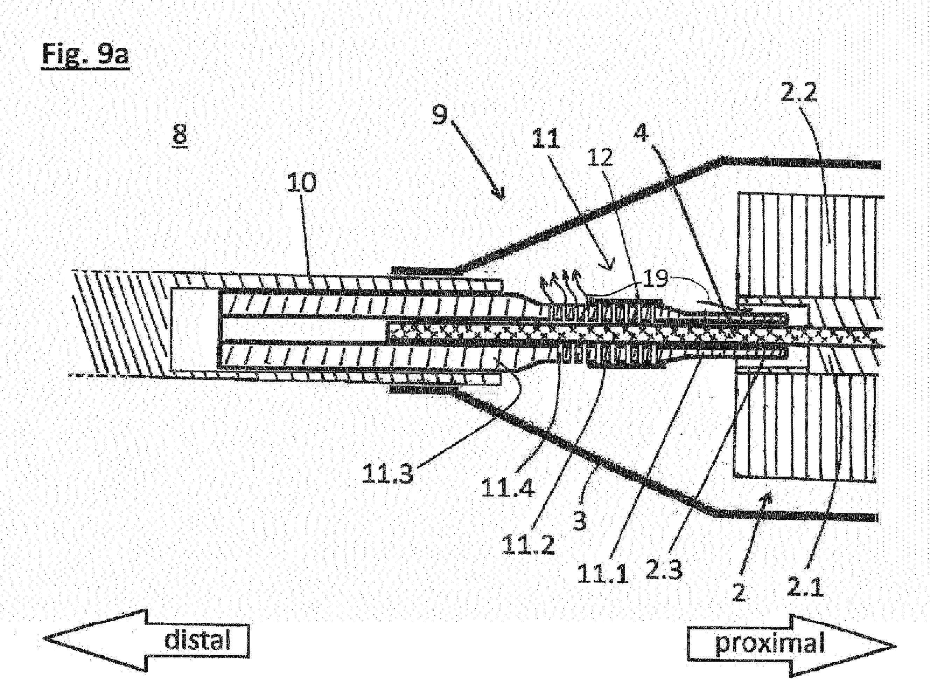

Figure 9a shows the catheter device 1 of Figure 8 with an additional flexible tube 12 provided around the pliable central section 11.2 of the drive shaft cover 11. The flexible tube 12 is designed as a shrink hose, covering part of the one or more opening 11.4. The flexible tube 12 is made of a polymer. The thickness of the flexible tube can be between 5 µm and 100 µm, preferably between 10 µm and 50 µm. The flexible tube 12 changes the flexibility of the pliable section. In order to avoid damage to the drive shaft 4 and ensure safe operation of the catheter device, thickness and material of the flexible tube as well as its position and length are chosen in accordance with the bending properties of the flexible housing 3 and the drive shaft to obtain optimal bending properties of the catheter device 1. The flexible tube thereby helps to avoid ingestion of heart tissue between the drive shaft cover and drive shaft.

In the Example from Figure 9a, the flexible tube 12 furthermore leaves a distal portion of the at least one opening 11.4 uncovered, thus providing a fluid path 19 through the hollow space, along the inside of the drive shaft cover 11 and through the uncovered openings 11.. This enables a flow of blood from the inside of the drive shaft cover 11 (where the drive shaft 4 is located) to the outside. This can help to prevent clogging of the device and can serve as a cooling mechanism.

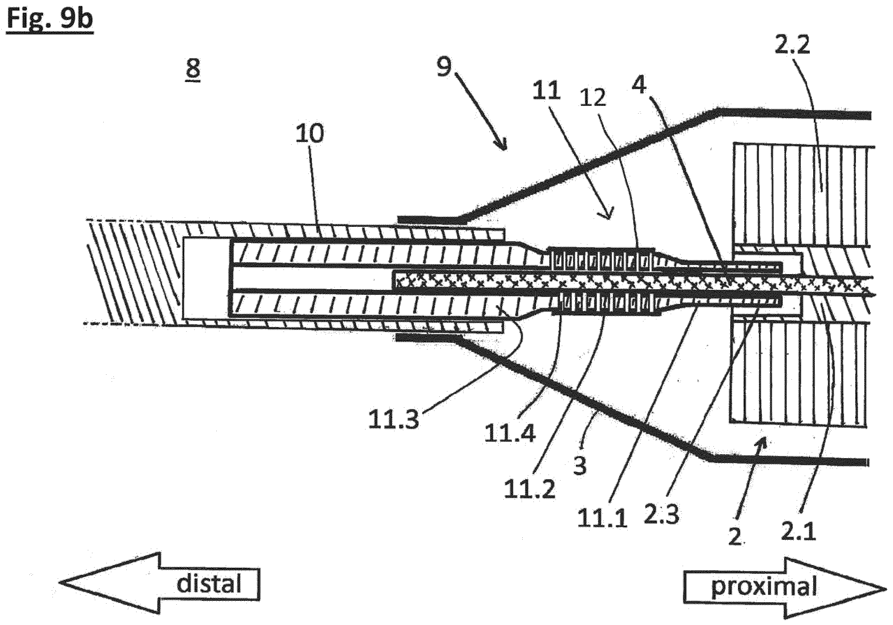

Figur 9b shows a setup which is similar to the one from Figure 9a. However, in the case of Figure 9b, the flexible tube 12 extends over the whole portion of the drive shaft cover 11, in which the openings 11.4 are provided. In this setup, where the flexible tube extends along the whole section having the openings 11.4, fluid communication between the inside of the drive shaft cover 11 and the outside of the flexible tube 12 can nonetheless be provided, for instance by having openings (not shown) in the flexible tube 12. Such openings in the flexible tube are not limited to have a specific geometry. In one embodiment, openings of the flexible tube 12 are chosen such that only a portion or a part of the opening or openings 11.4 in the drive shaft cover 11 is left uncovered. In particular, if several slits are provided in the drive shaft cover 11, each of the slits can be partially covered by the flexible tube 12. This way, the desired bending properties and the desired amount of fluid communication can be maintained, while reducing the risk of ingestion of heart tissue into the drive shaft cover 11 through the openings 11.4 of the drive shaft cover 11.

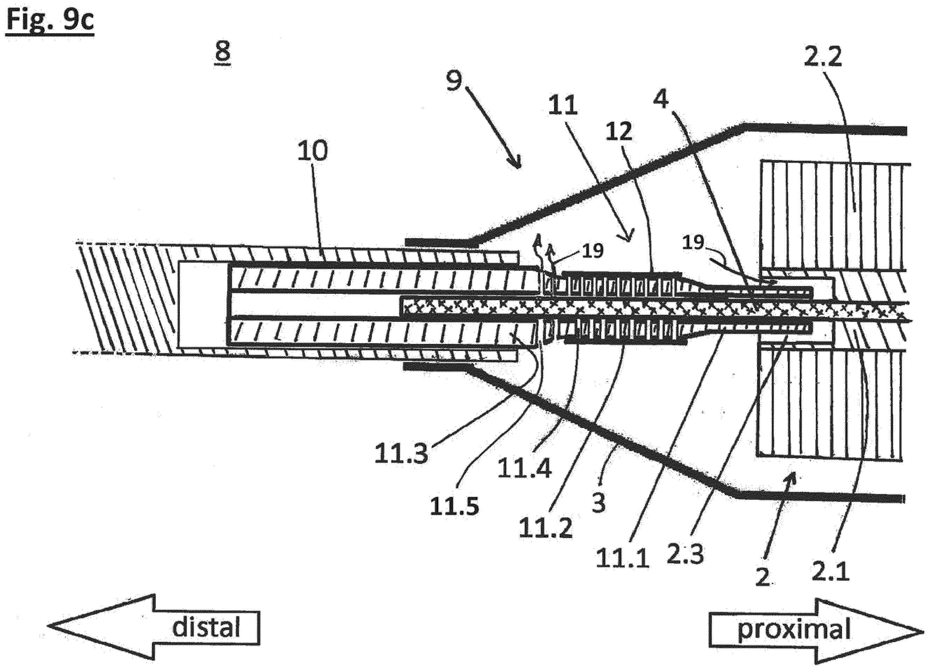

Figure 9c shows a setup that is similar to the one from Figures 9b in the sense that the flexible tube 12 extends over all of the openings 11.4. This way, the openings 11.4 can be designed for optimal bending properties and it is not necessary to adapt the design to prevent suction of tissue into the openings 11.4. A fluid path 19 similar to the one from Figure 9a is established by having additional venting holes 11.5 which are provided in the drive shaft cover 11, distally of the openings 11.4 of the pliable central section 11.2, and distally of the flexible tube 12. A setup of this type can have the further advantage that the bending properties can be tuned via the flexible tube 12 along the whole length of the pliable section. Thereby, the flexible tube can remain fully intact, yet a blood flow through the drive shaft cover 11 is possible. The venting holes 11.5 can be designed such that the region where they are located remains stiff, i.e. the venting holes can have a design that is different from the openings 11.4. The venting holes 11.5 are typically not limited to a specific shape. The venting holes 11.5 can be optimized for the intended blood flow and they can be optimized to avoid suction of tissue into the venting holes 11.5.

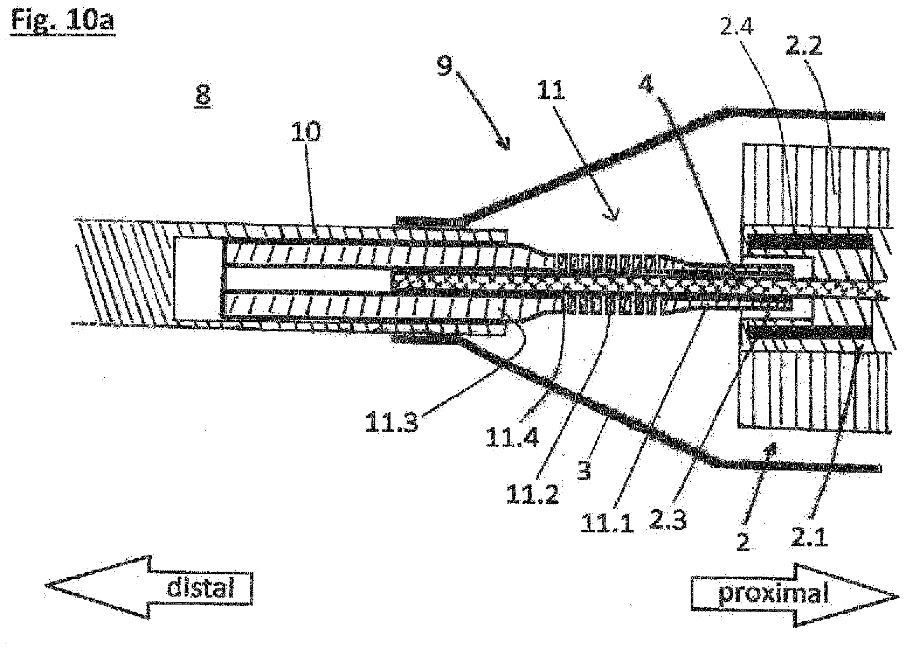

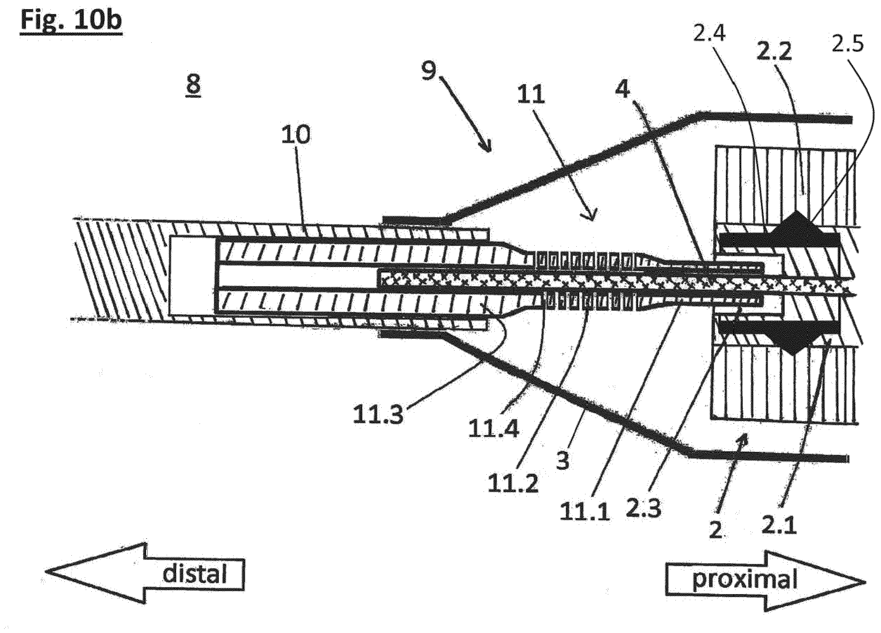

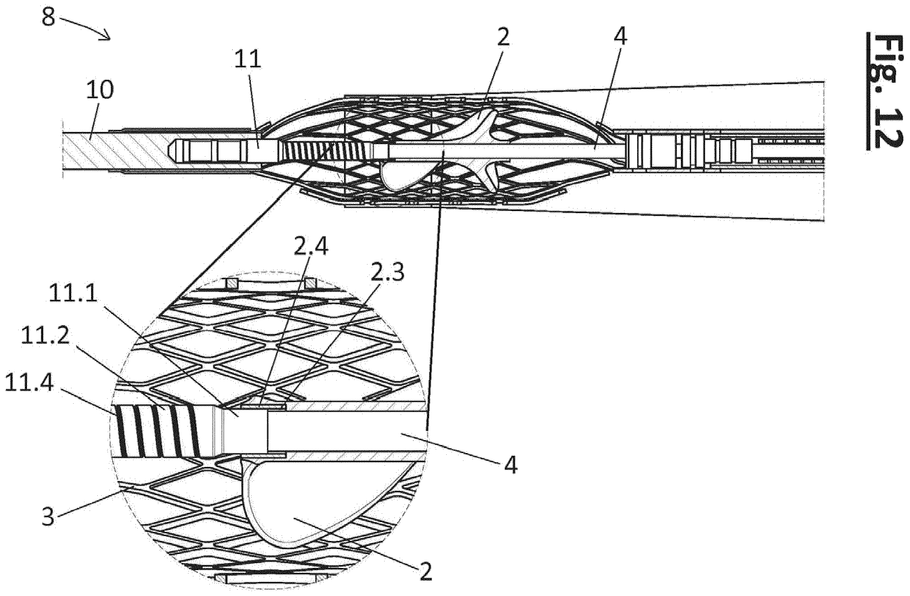

Figure 10a once again shows the catheter device 1 of Figure 8. This time, the rotor 2 is equipped with a stiffening element 2.4 surrounding the hollow space 2.3. The stiffening element 2.4 is a hollow cylinder which is embedded into the material of the rotor 2 and extends at least along a full length of the hollow space 2.3. Specifically, in the example shown in Figure 10, it is approximately twice as long as the hollow space 2.3, the stiffening element 2.4 having a length of for instance between 1.8 and 2.2 mm. In other possible embodiments it extends only over part of the length of the hollow space. The stiffening element 2.4 is used to prevent or reduce deformation of the rotor hub 2.1 during operation. An inner surface of the stiffening element 2.4 can also be left uncovered in an alternative embodiment (cf. Fig. 12). The stiffening element 2.4 may comprise microscopic and/or macroscopic structures to provide better attachment of the stiffening element to the rotor 2. These microscopic or macroscopic structures can for instance be designed as anchoring structures (cf. Fig. 10b) or indentations or holes, in particular through-holes, into which holes the material of the rotor 2 can penetrate (cf. Figs. 17 - 20). If holes are provided, they may have a diameter of for instance at least 0.05 mm to allow the material of the rotor to enter the holes. If a stiffening element 2.3 is provided, a radial gap within the hollow space 2.3, lying between the outside of the drive shaft cover and the rotor 2, can be made smaller. If no stiffening element 2.4 is provided, the radial gap might need to be larger in order to avoid touching of the parts due to possible ovalization of the rotor hub 2.1 during operation. The hollow space 2.3 should be present immediately after compression of the rotor 2, in order for the drive shaft cover 11 to be able to move back into position after being moved away or pulled out of the hollow space 2.3 during compression of the rotor (as described in the context of Fig. 7).

Figure 10b shows the catheter device 1 with the stiffening element 2.4. The stiffening element 2.4 comprises macroscopic anchoring elements 2.5 protruding radially outwards on two opposing sides of the stiffening element 2.4. The anchoring elements 2.5 are thereby located in areas where the blades 2.2 are attached to the hub 2.1 of the rotor 2. This way, it is possible to have anchoring elements 2.5 extending beyond the diameter of the hub 2.1 and into the material of the blades 2.2. The anchoring elements 2.5 are designed such that they still allow compression of the rotor 2 (i.e., folding of the rotor blades 2.2) as shown for instance in Fig. 7b, without damaging the rotor blades 2.2. In the present example, in order to allow the compression of the rotor, the anchoring elements 2.5 extend for example at most 1 mm or most 0.5 mm past the hub 2.1 and into the blades 2.2. The anchoring elements 2.5 can further comprise one or more recesses, indentations or undercuts into which the material of the rotor 2 may penetrate. The macroscopic protrusions can also be combined with holes and/or indentations.

The flexible tube 12 from Figures 9 a-c and the stiffening element 2.4, as shown for instance in Figures 10 a - c, can of course be combined in an advantageous embodiment of the catheter device according to this application. These embodiments are also compatible with the shorter drive shaft 4 as shown in Figure 8b.

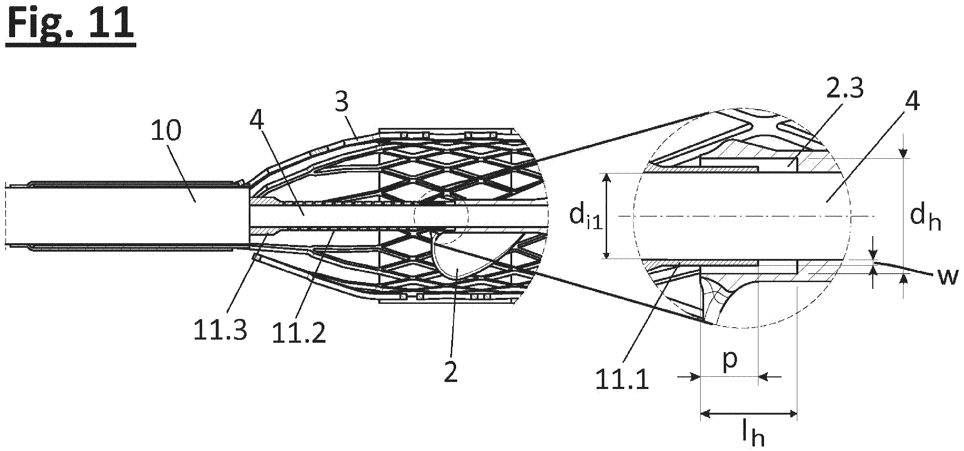

Figure 11 shows the catheter device 1 with a detailed view of the area around the hollow space 2.3. In the detailed view, the proximal section 11.1 of the drive shaft cover 11 can be seen as it penetrates into the hollow space 2.3.

The hollow space 2.3 has a length lₕ of between 0.9 mm and 1.1 mm. A penetration depth p of the proximal section 11.1 into the hollow space is between 0.3 mm and 0.7 mm, leaving some space between the proximal end of the drive shaft cover 11 and the rotor 2 to avoid touching of the parts.

Thereby, the penetration depth p is chosen such that lengthening of the housing 3 as shown for instance in Figures 7a and b can be tolerated. In particular, as the housing 3 is compressed, the distal bearing 9 and thus the drive shaft cover 11 are displaced in distal direction with respect to the rotor 2 and the drive shaft 4. This displacement is for instance equivalent to the change in length 3.2. The penetration depth p is chosen larger than said displacement in order to ensure that the drive shaft 4 remains inside the drive shaft cover 4 at all times, also during insertion of the catheter device into a patient, i.e., in the compressed state when the drive shaft cover 11 is moved away from the rotor 2 in distal direction.

Typically, a distance of at least 0.3 mm and at most 0.6 mm is provided in axial direction between the parts as an axial gap (lₕ-p). The axial gap allows for clearance under the expected bending loads occuring during use of the pump.

A radial gap between the parts of the rotor 2 radially delimiting the hollow space 2.3 and an outer surface of the drive shaft cover is between 0.07 mm and 0.13 mm to avoid touching of the parts upon for example ovalization of the rotor 2, i.e., ovalization of the rotor hub 2.1. It is advantageous to keep a diameter dₕ of the cylindrical hollow space 2.3 as small as possible. A constraint for an inner diameter dᵢ₁ of the proximal section 11.1 of the drive shaft cover 11 is however given by the diameter of the drive shaft 4. A wall thickness w of the proximal section 11.1 of the drive shaft cover 11 is therefore chosen to be as small as possible. In this example, the wall thickness w is between 0.05 mm and 0.07 mm. Given a typical diameter of the drive shaft 4, the diameter dₕ of the hollow space may for instance be between 1.1mm and 1.3 mm to achieve a radial gap given by dₕ-w-dᵢ₁, having the above-described dimensions.

An inner diameter dᵢ₁ of the drive shaft cover in the proximal section 11.1 is thereby chosen in accordance with an outer diameter of the drive shaft 4 to provide good bearing of the drive shaft 4 while enabling rotation of the drive shaft 4 without unnecessary wear and tear.

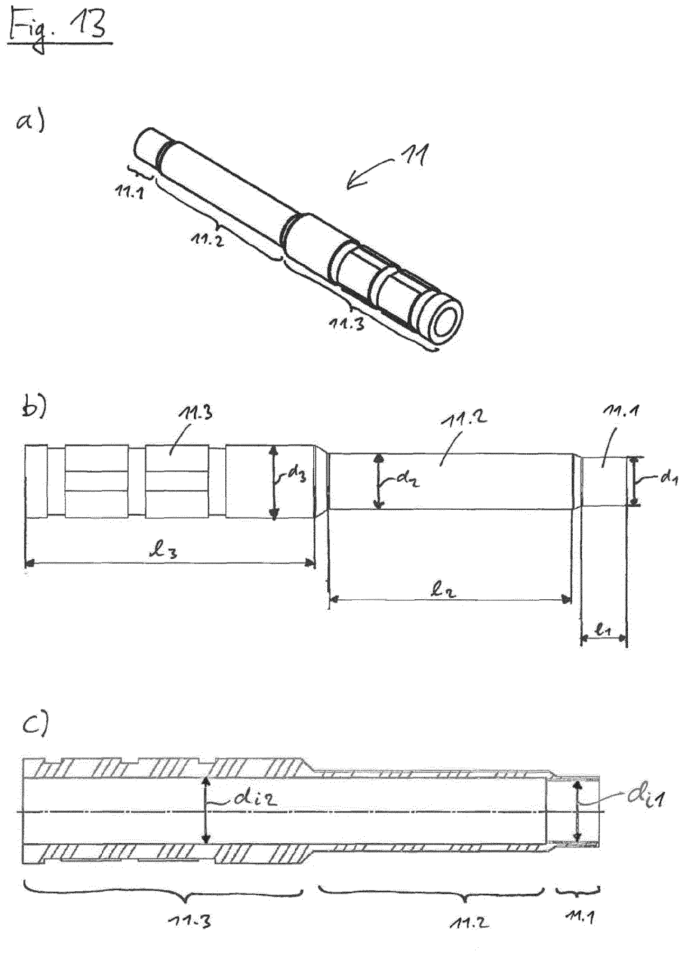

Some of the proximal section 11.1 remains outside of the hollow space 2.3. Thus, the diameter of the drive shaft cover 11 increases at a distance from a distal end of the rotor 2, depending on the length of the proximal section, for instance at least 0.3 mm away from the distal end of the rotor 2 (cf. Fig. 13b).

Figure 12 shows an enlarged view of a section of the catheter device 1 with the stiffening element 2.4 provided around the hollow space. Here, the stiffening element 2.4 delimits the hollow space 2.3, having no additional material of the rotor on the inside of hollow cylinder that is the stiffening element 2.4. The stiffening element is made of a bio-compatible material. It can comprise MP35N and/or Nitinol and/or stainless steel and/or ceramics. It has a wall thickness of between 0.04 mm and 0.07 mm. It may have indentations on the outside, in order to better engage with the material of the rotor.

Figures 13 a-c show three different views of the drive shaft cover 11.

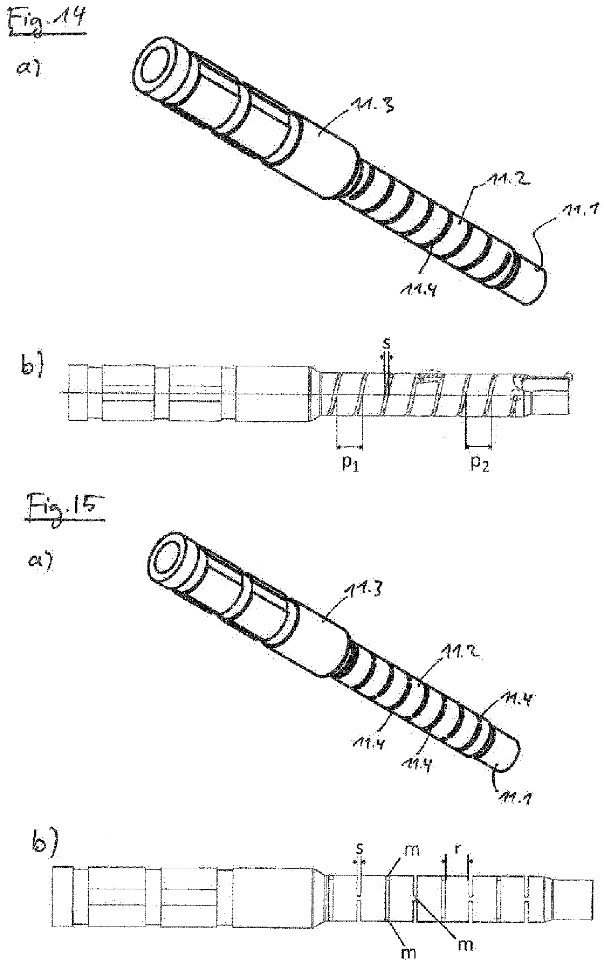

Figure 13 a shows a perspective view. The proximal section 11.1, having the smallest diameter, the central section 11.2, having an increased diameter, and the distal section 11.3, having the largest diameter can be seen. The central section is typically pliable. Pliability can be provided by having openings or slits in the central section (cf. Figs. 14 -16). The pliable central section 11.2 can thereby be limp or flexible with a memory-effect, in the sense that the pliable section 11.2 regains its original shape after deformation. For better visibility, no slits are shown in Figures 13 a-c. A device as shown in Figure 13, without any slits, can be provided as a single piece and the slits can be cut into the single piece for manufacturing the drive shaft cover 11, using for instance a laser.

The slits 11.4 can be arranged such that a so-called hypotube-design is achieved. Examples of such hypotube-designs are for instance shown in Figures 14a-16b.

In the distal section, which is provided inside the end part 10, indentations are provided on the outside of the drive shaft cover. This way, a material of the end part, such as a polymer, may enter the indentations and thus form a particularly stable connection with the distal section 11.3.

In Figure 13 b, a schematic side view is shown. Outer diameter and length of each section are visible. The proximal section 11.1 has a first length l₁ which is between 0.9 mm and 1.1 mm. If it penetrates the hollow space 2.3 as described above, by between 0.3 mm and 0.7 mm, a remainder of the proximal section 11.1 remains outside of the hollow space 2.3. The length of the proximal section may be chosen such that a length of the remainder of the proximal section 11.1 remaining outside of the hollow space is the same as the length of the axial gap. An outer diameter d₁ can be for instance between 0.9 mm and 1.1 mm, depending on a diameter of the drive shaft 4.

Distally of the proximal section 11.1, a central section 11.2 is provided. The outer diameter d₂ of the central section 11.2 is between 0.14 mm and 0.3 mm larger than d₁. The length l₂ of the central section 11.2 can be for instance between 5 and 8 mm.

The distal section 11.3 has a length 13 which can be between 5 and 8 mm and an outer diameter d₃ which is larger than d₂. The outer diameter d₃ can be for instance between 1.25 mm and 1.6 mm. Furthermore, on the outer surface of the distal section 11.3, axial and circumferential grooves are realized for a solid connection to the end part 10.

In Figure 13 c, a cut through the drive shaft cover 11 is shown, exposing the inside of the drive shaft cover 11 where the drive shaft 4 is located during operation. An inner diameter dᵢ₁ at the proximal end of the drive shaft cover 11 is smaller than an inner diameter dᵢ₂ at its distal end. The diameter is thereby changed in a smoothened step, between the proximal section 11.1 and the central section 11.2, the inner diameter being kept constant throughout the central section 11.2 and the distal section 11.3. A difference in diameter between dᵢ₁ and dᵢ₂ is between 0.02 mm and 0.12 mm. Having a smooth transition between the different inner diameters helps to avoid wear of the drive shaft.

Figures 14 a and b show different views of the drive shaft cover 11, the drive shaft cover 11 having a pliable central section 11.2.

Figure 14 a shows a perspective view. An opening 11.4 designed as a spiral slit extends essentially over the whole length of the central section 11.2. The slit connects an inside of the drive shaft cover 11 to an outside of the drive shaft cover 11 such that the remaining material of the central section 11.2 forms a spiral sleeve.

Figure 14 b shows a corresponding side view. The spiral slit can have a width s of for instance between 0.005 mm and 0.2 mm, preferably between 0.025 mm and 0.1 mm. The width s of the slit can be tuned to achieve the desired bending properties. It can also be chosen in such a way that blood circulation through the slit is possible. Edges of the slit can be rounded to avoid wear on the drive shaft, tissue, or a flexible tube.

The pitch of the spiral can also be chosen according to the desired bending properties. The pitch can therefore change along a length of the spiral, having a first pitch associated with a length first length p₁ at the distal end of the spiral and a second pitch associated with a second length p₂ at the proximal end of the spiral, wherein p₁ is for example larger than p₂. The pitch may also be kept constant in an embodiment of the drive shaft cover.

The pitch may be for instance between 0.5 mm and 0.8 mm.

The slit can be cut into the drive shaft cover 11 using a laser.

Figures 15 a and b show the same views as Figs. 14 a and b, but for a different slit arrangement. In the case of Figure 15, several slits extending tangentially in the pliable central section 11.2 are provided. In fact, the course of the slits does not have an axial component, i.e., they run circumferentially with no pitch. It is however possible to provide a multitude of slits with an axial component.

In the setup shown in Figure 15, several pairs of slits are provided at in the central section 11.2, each pair of slits comprising two slits arranged at the same height of the central section 11.2 and running almost halfway around the central section, leaving two bridges m on opposing sides, each bridge m having a width of for instance between 0.05 mm and 0.2 mm. The pairs of slits are arranged at a distance r from one another.

In one embodiment, the width of the slits arranged as in Figure 15 is the same as that of the slits as shown in Figure 14. Due to the circumferential arrangements, the slits may however also be wider than the slits shown in Figure 14. The pairs of slits are arranged at a distance of between 0.3 mm and 1 mm from one another, the bridges m of the pairs of slits being arranged at different angles from one pair of slits to the next. In the case of Figure 15, at 0° and 180° for the first pair of slits, and at 90° and 270° for the second pair of slits and so forth in an alternating fashion.

The width s of the slits can be the same as in the case of the spiral slit and they can also be cut using a laser.

The distance r between the slits, as indicated in Figure 15 b, corresponding to a width of the material between the slits, can be larger than the width s of the slits in one embodiment. It is however also possible, to make the distance r between the slits small, in an exemplary embodiment even smaller than the width of the slits, in order to allow bending of the pliable central section 11.2 through deformation of the material of width r between the slits.

In particular in a setup of this type, where the material of width r between the slits can be deformed, it is also possible to have more than two slits arranged at the same height, for instance three slits at the same height, each slit running around less than a third of the circumference, in this case having three bridges, for instance of the above-described width. Then, bending of the pliable section can be enabled via deformation of the material of width r between the slits, rather than deformation of the bridges. It is of course also possible to have more than three slits and three bridges, such as for instance four slits and four bridges.

Figure 16 a shows a perspective view of the drive shaft cover 11, with a pliable central section 11.2 having several openings 11.4. The openings 11.4 are designed as slits with a tangential component and an axial component. All slits have the same pitch. The slits are intertwined spiral sections, each slit running 240° around the central section and each slit having through-holes at both ends. In each case, three slits of the above-described type are provided at the same height, starting at 120° from one another, on the circumference of the drive shaft cover. The width s of the slits can be the same as in the case of Figures 14 and 15. The through-holes which are provided at both ends of each slit have a cross section with a geometry that may be optimized for preventing tearing or rupturing of the drive shaft cover upon strong deformation. They may be for example circular or drop-shaped. They may have a diameter or edge length that is larger than the width of the slits, in particular they may have a diameter of edge length of for instance between 0.05 mm and 2 mm.

Figure 16 b shows a portion of the pliable central section 11.2 of the drive shaft cover 11. Thereby, the slits 11.4 run all the way around the drive shaft cover, resulting in several rings or segments. The design allows for full flexibility within the constraints of the laser cut width. Two segments are shown in the Figure. The cut structure can be repeated axially at a given distance, resulting in more segments of the shown type. The segments are held together by an undercut design: A left segment has a recess and a right segment has a protrusion lying in the recess of the left segment, thus connecting the two segments, similar to pieces of a jigsaw-puzzle. Several pairs of recesses and protrusions of this type are arranged on the circumference of the segments, so that the segments do not disengage as they are moved with respect to each other. For instance at least 2 recess-protrusion pairs or at least 3 recess-protrusion pairs or at least 4 recess-protrusion pairs are provided. While the segments are connected in said fashion, no material bridges are provided between the segments.

The slit 11.4 is wide enough to provide play between the two segments, rendering the section pliable, more specifically, rendering the section limp. The section has a minimal bending radius that is limited by the play provided by the slit 11.4, i.e., bending is only possible to a certain degree, until the segments abut.

A restoring force for straightening the drive shaft cover after it has been bent can be provided for instance by providing the flexible tube 12 around the pliable section 11.2 of the drive shaft cover 11.

Figures 17 to 20 show different embodiments of the stiffening element 2.4, in each case providing a side view displayed in subfigure a) and a perspective view displayed in subfigure b).

All of the stiffening elements 2.4 shown in Figures 17 to 20 may have a length lₛ that is chosen in accordance with the length of the hollow space 2.3 of the rotor 2. The length lₛ may be for instance twice the length of the hollow space 2.3, for instance between 1.8 mm and 2.2 mm.

The stiffening elements 2.4 may be made of a bio-compatible material. They may comprise one or more of MP35N, 35NLT, Nitinol, stainless steel (in particular medical grade stainless steel), and ceramics.

An inner diameter dᵢₛ of the stiffening element 2.4, in the case of each of the embodiments shown in Figures 17-20, may be chosen to at least the diameter of the hollow space 2.3.

An outer diameter dₒₛ may be chosen to be smaller than an outer diameter of the hub 2.1 of the rotor.

A wall thickness of the stiffening element, may, in each case, be for instance at least 0.03 mm, preferably at least 0.04 mm and/or at most 0.08 mm, preferably at most 0.07 mm.

In the case of Figures 17 a and b, the stiffening element is designed as a tube, having through-holes near one end of the stiffening element. The through-holes may be provided in a section of the rotor lying proximally of the hollow space 2.3, the portion of the stiffening element 2.4 without any through-holes extending along the hollow space 2.3, in particular delimiting the hollow space. I.e., in this case the inside the portion without any holes may remain exposed (cf. Fig. 12). In this case, dᵢₛ is then equal to the diameter of the hollow space 2.3.

The section having through-holes, which can extend proximally of the hollow space 2.3 can be completely surrounded by the material of the rotor. The material of the rotor may penetrate through the through-holes, enabling a particularly reliable connection between the rotor 2 and the stiffening element 2.4. A cross section of the holes is circular. It is however not limited to this specific geometry. They may be circular or polygonal. The holes have a diameter of between 0.03 mm and 0.5 mm.

Figures 18 a and b show an embodiment of the stiffening element 2.4, wherein through-holes are provided along the full length of the stiffening element 2.4. In this case, dᵢₛ can for example be chosen to be larger than the diameter of the hollow space 2.3. The stiffening element 2.3 can then be completely surrounded by the material of the rotor 2 or the rotor hub 2.1. I.e., a thin layer of material pertaining to the hub 2.1 of the rotor can be provided on the inside of the stiffening element 2.4 in the region of the hollow space 2.3 (cf. Fig. 10). This way, the stiffening element 2.4 is not exposed. The holes have a diameter of between 0.3 mm and 0.5 mm.

Figures 19 a and b show an embodiment of the stiffening element 2.4 which is similar to the one shown in Figure 17, i.e. suitable to be used in a setup as shown for instance in Fig. 10. The through-holes provided in the stiffening element 2.4 of Figure 19 are however smaller and have a diameter of between 0.02 mm and 0.1 mm.

Figures 20 a and b show an embodiment of the stiffening element 2.4, the stiffening element being designed as stent-like structure. The stent-like structure comprises three rings, one at each end and one being provided centrally.

The invention is defined by the claims. The following aspects may be useful for understanding the invention and form part of the disclosure.
1. A catheter device (1), comprising:
   a rotor (2,2') located at the distal end region (8) of the catheter device (1);
   a drive shaft (4) extending from a driving region (16) of the catheter device (1) to the distal end region (8) of the catheter device (1);
   a distal bearing (9) for bearing a distal end of the drive shaft (4); and wherein
   the distal bearing (9) comprises a heat conducting part (13) configured to enable heat transfer away from the distal bearing.
2. A catheter device according to aspect 1, characterized in that the heat conducting part (13) is designed as a tube surrounding the drive shaft.
3. A catheter device (1) according to any one of aspects 1 or 2, characterized in that the drive shaft (4) comprises a cavity extending axially with the drive shaft and wherein the drive shaft comprises a plurality of coaxial windings which run spirally around the cavity of the drive shaft, the windings within different coaxial layers having opposite winding directions and in that the outer diameter of the drive shaft lies in a range of about 0.4 mm to about 2 mm, preferably comprising a reinforcement element which is provided sectionally in the cavity of the drive shaft (4) in the distal end region.
4. A catheter device (1) according to any one of aspects 1 to 3, characterized in that the heat conducting part (13) extends out of the distal bearing (9), into an area which is configured to be brought in contact with the fluid, enabling heat transfer from the distal bearing (9) to the fluid.
5. A catheter device (1) according to any one of aspects 1 to 4, characterized in that the distal bearing (9) comprises a polymer end part (10) or the distal bearing (9) comprises a polymer end part which comprises a region which is designed as a pigtail (10.2).
6. A catheter device (1) according to any one of aspects 1 to 5, characterized in that the heat conducting part (13) is made of a medical grade stainless steel, preferably made of 1.4441 stainless steel.
7. A catheter device (1) according to any one of aspects 1 to 6, characterized in that an inner diameter of the heat conducting part (13) designed as a tube is between 0.5 mm and 2.6 mm and/or in that the heat conducting part (13) has a thickness between 0.05 mm and 0.5 mm.
8. A catheter device (1) according to any one of aspects 1 to 7, characterized in that a spiral sleeve (14) with a winding is arranged within the distal bearing (9), for rotatably mounting the distal end of the drive shaft (4) inside the spiral sleeve (14), such that the spiral sleeve (14) lies at least in part inside the heat conducting part (13) designed as a tube and/or such that a portion of the spiral sleeve (14) is in direct contact with a portion of the inner side (13') of the heat conducting part (13)
9. A catheter device (1) according to any one of aspects 1 to 8, characterized in that a spiral sleeve (14) with a winding is arranged within the distal bearing (9), for rotatably mounting the distal end of the drive shaft (4) inside the spiral sleeve (14), such that a portion of the spiral sleeve (14) and a portion of the heat conducting part (13) are only separated by a thin flexible tube (12,12') which is provided around a portion of the outside of the spiral sleeve, wherein the flexible tube (12,12') is preferably designed as a shrink hose.
10. A catheter device (1) according to aspect 8 or 9, characterized in that the spiral sleeve (14) is made of flat tape (14.1).
11. A catheter device (1) according to any one of aspects 1 to 10, characterized in that a portion of the outer side (13") of the heat conducting part (13) which is configured to be brought in contact with the fluid is smooth, preferably with a ten-point mean roughness of R_{z}≤1.2 µm, and in that an inner side (13') of the heat conducting part (13) is rough to facilitate gluing the spiral sleeve (14) to the inner side (13') of the heat conducting part (13), the inner side (13') of the heat conducting part or tube (13) preferably having an arithmetic average surface roughness of Rₐ≥0.8 µm.
12. A catheter device (1) according to aspect 11, characterized in that a further portion of the outer side (13") of the heat conducting part or tube (13), which is configured to lie inside the polymer end part (10), is roughened, preferably having an arithmetic average surface roughness of Rₐ≥0.8 µm.
13. A catheter device (1) according to any one of aspects 8 to 12, characterized in that both ends of the spiral sleeve (14) are face ground and all edges of both ends are rounded and smooth, preferably with a ten-point mean roughness of R₂≤2 µm.
14. A catheter device (1) according to any one of aspects 8 to 13, characterized in that an inner diameter of the spiral sleeve (14) is between 0.4 mm and 2.1 mm, and in that the spiral sleeve has a thickness between 0.05 mm to 0.4 mm.
15. A catheter device (1) according to any one of aspects 8 to 14, wherein the rotor (2) and the drive shaft (4) are configured to rotate in a rotating direction (4.1) such that a flow of fluid in a proximal direction is effected, if the catheter device (1) is brought in contact with a fluid, characterized in that, when looking along the drive shaft towards a distal end of the drive shaft, the winding direction of the spiral sleeve (14) from a proximal end of the spiral sleeve (14) to a distal end of the spiral sleeve (14), is the opposite direction of the rotating direction (4.1) of the rotor (2) and the drive shaft (4), when looking along the drive shaft (4) towards a distal end of the drive shaft (4).
16. A catheter device (1) according to any one of aspects 8 to 15, characterized in that the spiral sleeve (14) is made out of MP35N^{®}, 35NLT^{®}, or ceramics.
17. A catheter device (1) according to any one of aspects 1 to 16, designed as an expandable pump, characterized in that a cannula (15) is provided around a portion of the drive shaft which lies in the vicinity of the rotor (2) and in that the rotor (2) is located in a housing (3), the housing (3) and the rotor (2) being configured to be transferred at least in part into the cannula (15), wherein the housing (3) and the rotor (2) are compressed at least along a radial direction extending transversely to a longitudinal direction, from an expanded state into a compressed state.
18. A catheter device (1) according to aspect 17, wherein, upon application of a force at the proximal end of the catheter and/or compression of the housing (3) and the rotor (2), a relative motion of the drive shaft (4) with respect to the distal bearing (9) is effected, and wherein the drive shaft (4) and the distal bearing (9) are configured such that the distal end of the drive shaft (4) remains within the distal bearing (9) or within the heat conducting part (13) designed as a tube or within the spiral sleeve (14) when the housing (3) and the rotor (2) are compressed.
19. A catheter (1) device according to any one of aspects 1 to 18, characterized in that a hub (2.1) pertaining to the rotor (2) extends less than 0.5 mm past the rotor blades (2.2) towards the distal end of the catheter device, preferably less than 0.1 mm.
20. A catheter device (1), comprising:
   a rotor (2) located at the distal end region of the catheter device (1);
   a drive shaft (4) extending from a driving region (16) of the catheter device (1) to the distal end region (8) of the catheter device;
   a distal bearing (9) for bearing a distal end of the drive shaft; and wherein
   the distal bearing (9) comprises a spiral sleeve (14) with a winding, configured for rotatably mounting the distal end of the drive shaft (4) inside the spiral sleeve (14).
21. A catheter device (1) according to aspect 20, characterized in that the spiral sleeve (14) is made of flat tape (14.1).
22. A catheter device (1) according to any one of aspects 20 or 21, characterized in that the drive shaft (4) comprises a cavity extending axially with the drive shaft (4) and wherein the drive shaft (4) comprises a plurality of coaxial windings which run spirally around the cavity of the drive shaft (4), the windings within different coaxial layers having opposite winding directions.and in that the outer diameter of the drive shaft lies in a range of about 0.4 mm to about 2 mm, preferably comprising a reinforcement element which is provided sectionally in the cavity of the drive shaft (4) in the distal end region.
23. A catheter device (1) according to any one of aspects 20 to 22 , characterized in that both ends of the spiral sleeve (14) are face ground and all edges of both ends are rounded and smooth, preferably with a ten-point mean roughness of R_{z}≤2 µm.
24. A catheter device according to any one of aspects 20 to 23, characterized in that a flexible tube (12, 12') is provided around a portion of the outside of the spiral sleeve, wherein the flexible tube is preferably designed as a shrink hose.
25. A catheter device (4) according to any one of aspects 20 to 24, wherein the rotor (2) and the drive shaft (4) are configured to rotate in a rotating direction (4.1) such that a proximally directed flow of fluid is effected, if the catheter device (1) is brought in contact with a fluid, characterized in that, when looking along the drive shaft (4) towards a distal end of the drive shaft, the winding direction of the spiral sleeve (14) from a proximal end of the spiral sleeve (14) to a distal end of the spiral sleeve (14), is the opposite direction of the rotating direction (4.1) of the rotor (2) and the drive shaft (4), when looking along the drive shaft towards a distal end of the drive shaft.
26. A catheter device (1) according to any one of aspects 20 to 25, characterized in that the spiral sleeve (14) is made out of MP35N^{®}, 35NLT^{®}, or ceramics.
27. A catheter device (1) according to any one of aspects 20 to 26, characterized in that an inner diameter of the spiral sleeve (14) is between 0.4 mm and 2.1 mm and in that the spiral sleeve has a thickness between 0.05 mm to 0.4 mm.
28. A catheter device (1) according to any one of aspects 20 to 27, characterized in that the spiral sleeve (14) and/or the flexible tube (12,12') is at least in part in contact with a heat conducting part (13), the heat conducting part (13) being configured to enable heat transfer away from the distal bearing (9) and/or the spiral sleeve (14).
29. A catheter device according to aspect 28, characterized in that the heat conducting part (13) is designed as a tube surrounding a portion of the spiral sleeve (14).
30. A catheter device according to aspect 28 or 29, characterized in that the heat conducting part or tube (13) extends out of the distal bearing, into an area which is configured to be brought in contact with a fluid, enabling heat transfer from the distal bearing (9) to the fluid.
31. A catheter device (1) according to any one of aspects aspect 20 to 30, characterized in that the distal bearing (9) comprises a polymer end part (10) or the distal bearing (9) comprises a polymer end part which comprises a region which is designed as a pigtail (10.2).
32. A catheter device (1) according to any one of aspects 28 to 31, characterized in that a portion of the outer side (13") of the heat conducting part (13) which is configured to be brought in contact with the fluid is smooth, preferably with a ten-point mean roughness of R_{z}≤1.2 µm, and in that an inner side (13') of the heat conducting part (13) is rough to facilitate gluing the spiral sleeve (14) to the inner side (13') of the heat conducting part (13), the inner side (13') of the heat conducting part or tube (13) preferably having an arithmetic average surface roughness of Rₐ≥0.8 µm.
33. A catheter device according to aspect 32, characterized in that a further portion of the outer side (13") of the heat conducting part or tube (13) which is configured to lie inside the polymer end part is roughened, preferably having an arithmetic average surface roughness of Rₐ≥0.8 µm.
34. A catheter device according to any one of aspects 28 to 33, characterized in that an inner diameter of the heat conducting part (13) designed as a tube is between 0.5 mm and 2.6 mm and/or in that the heat conducting part has a thickness between 0.05 mm and 0.5 mm.
35. A catheter device according to any one of aspects 28 to 34, characterized in that the heat conducting part (13) is made of a medical grade stainless steel, preferably made of 1.4441 stainless steel.
36. A catheter device (1) according to any one of aspects 20 to 35, designed as an expandable pump, characterized in that a cannula is provided around a portion of the drive shaft (4) which lies in the vicinity of the rotor (2) and in that the rotor (2) is located in a housing (3), the housing (3) and the rotor (2) being configured to be transferred at least in part into the cannula (15), wherein the housing (3) and the rotor (2) are compressed at least along a radial direction extending transversely to a longitudinal direction, from an expanded state into a compressed state.
37. A catheter device (1) according to any one of aspects 20 to 36, wherein, upon application of a force at the proximal end of the catheter and/or compression of the housing and the rotor, a relative motion of the drive shaft (4) with respect to the distal bearing (9) is effected, and wherein the drive shaft and the distal bearing are configured such that the distal end of the drive shaft remains within the spiral sleeve (14) when the housing (3) and the rotor (2) are compressed.
38. A catheter device (1) according to any one of aspects 20 to 37, characterized in that a hub (2.1) pertaining to the rotor (2) extends less than 0.5 mm past the rotor blades (2.2) towards the distal end of the catheter device, preferably less than 0.1 mm.
39. A catheter device (1), comprising:
   a rotor (2) located at the distal end region of the catheter device (1);
   a drive shaft (4) extending from a driving region (16) of the catheter device (1) to the distal end region (8) of the catheter device;
   a distal bearing (9) for bearing a distal end of the drive shaft; wherein
   the distal bearing (9) comprises a spiral sleeve (14) with a winding, configured for rotatably mounting the distal end of the drive shaft (4) inside the spiral sleeve (14);
   and wherein the spiral sleeve (14) or a flexible tube (12, 12'), which is provided around a portion of the outside of the spiral sleeve, is at least in part in contact, with a heat conducting part (13), the heat conducting part (13) being configured to enable heat transfer away from the distal bearing (9) and/or the spiral sleeve (14).
40. A catheter device (1), comprising
   - a drive shaft (4) extending from a driving region (16) of the catheter device (1) to a distal end region (8) of the catheter device (1),
   - a rotor (2) which is attached to the drive shaft (4) in the distal end region (8),
   - a distal bearing (9) for bearing a distal end of the drive shaft (4), wherein the distal bearing comprises a drive shaft cover (11), the drive shaft cover (11) being configured to cover a section of the drive shaft (4), which section extends distally of the rotor (2), characterized in that
   on a distal side of the rotor (2), a radially inner part of the rotor (2) is recessed with respect to radially outer parts of the rotor (2) to form a hollow space (2.3) surrounding the drive shaft (4), wherein a proximal end of the drive shaft cover (11) lies in said hollow space (2.3).
41. The catheter device (1) according to aspect 40, characterized in that a diameter of the hollow space (2.3) is at least 0.5 mm and/or at most 2 mm; and/or in that a length of the hollow space is at least 0.5 mm and/or at most 2.5 mm.
42. The catheter device (1) according to any of aspects 40-41, designed as an expandable pump, wherein the rotor (2) is located in a housing (3), the housing (3) and the rotor (2) being configured to be compressed at least along a radial direction extending transversely to a longitudinal direction, from an expanded state into a compressed state, and wherein upon compression of the housing (3), a relative motion of the rotor (2) with respect to the distal bearing (9) is effected, and wherein a penetration depth (p) of the drive shaft cover (11) over which it extends axially into the hollow space (2.3) is chosen such that the proximal end of the drive shaft cover (11) remains within the hollow space (2.3) in the compressed state.
43. The catheter device (1) according to any of aspects 40-42, characterized in that the penetration depth (p) of the drive shaft cover, over which it extends into the hollow space (2.3), is at least 0.3 mm and/or at most 2.2 mm.
44. The catheter device (1) according to any of aspects 40-43, characterized in that a wall thickness of a portion of the drive shaft cover (11) extending into the hollow space (2.3) is at least 0.03 mm and/or at most 0.3 mm, preferably at most 0.08 mm.
45. The catheter device (1) according to any of aspects 40-44, characterized in that an outer diameter of a proximal section (11.1) of the drive shaft cover (11) is smaller than an outer diameter of a section of the drive shaft cover (11) lying distally thereof, wherein a portion of the proximal section (11.1) extends into the hollow space (2.3), the outer diameter of the proximal section (11.1) of the drive shaft cover (11) preferably being at least 0.1 mm smaller than the outer diameter of the section of the drive shaft cover (11) lying distally thereof and/or at most 0.6 mm smaller than the outer diameter of the section of the drive shaft cover (11) lying distally thereof.
46. The catheter device (1) according to aspect 45, characterized in that the proximal section (11.1) has a length of at least 0.6 mm and/or at most 2 mm.
47. The catheter device (1) according to any of aspects 40-46, characterized in that a radial gap which is formed inside the hollow space (2.3), between the drive shaft cover (11) and the rotor (2), has a gap size of at least 0.01 mm and/or at most 0.2 mm; and/or characterized in that an axial gap of at least 0.2 mm and/or at most 1.5 mm remains between the proximal end of the drive shaft cover (11) and a hub (2.1) of the rotor (2).
48. The catheter device (1) according to any of aspects 40-47, characterized in that the rotor (2) comprises a stiffening element (2.4) surrounding the hollow space (2.3).
49. The catheter device (1) according to any of aspects 40-48, characterized in that the distal bearing (9) comprises an end part (10), wherein a distal end of the drive shaft cover (11) lies within the end part (10), the drive shaft cover (11) preferably comprising a distal section (11.3) with a diameter that is larger than a diameter of a section of the drive shaft cover (11) lying proximally thereof, wherein said distal section (11.3) lies in part inside the end part (10).
50. The catheter device (1) according to any of aspects 40-49, characterized in that the drive shaft cover (11) comprises a pliable section (11.2), the pliable section (11.2) preferably being arranged between a distal end of the rotor (2) and a proximal end of an end part (10) of the distal bearing (9).
51. The catheter device (1) according to any of aspects 40-50, wherein the pliable section (11.2) is provided by having at least one opening (11.4) in the drive shaft cover (11) in said pliable section (11.2), the at least one opening (11.4) connecting an inside of the drive shaft cover (11) to an outside of the drive shaft cover (11), the at least one opening (11.4) preferably comprising one or more slits.
52. The catheter device (1) according to aspect 51, characterized in that a flexible tube (12) is provided around the pliable section (11.2) of the drive shaft cover (11), covering the at least one opening (11.4) at least in part.
53. The catheter device (1) according to aspect 52, characterized in that the flexible tube (12) leaves a distal portion of the at least one opening (11.4) uncovered and/or in that the flexible tube (12) comprises one or more holes to allow fluid communication with a portion of the at least one opening (11.4) and/or in that the drive shaft cover (11) comprises one or more venting holes (11.4) to allow fluid communication between the inside of the drive shaft cover (11) and the outside of the drive shaft cover (11).
54. The catheter device (1) according to any of aspects 40-53, characterized in that an inner diameter of the drive shaft cover (11) at the proximal end of the drive shaft cover (11) is reduced with respect to an inner diameter of the drive shaft cover (11) at the distal end of the drive shaft cover (11).
55. The catheter device (1) according to any of aspects 40-54, characterized in that the drive shaft cover (11) comprises 35NLT and/or ceramics and/or a diamond-like-carbon coating and/or in that the drive shaft cover is manufactured from a single piece.

### List of reference numerals

- 1: Catheter Device
- 2: Rotor
- 2': Rotor (compressed state)
- 2.1: Hub
- 2.2: Rotor blade
- 2.3: Hollow space
- 2.4: Stiffening element
- 2.5: Anchoring element
- 3: Housing
- 3': Housing (compressed state)
- 3.1: Length of the housing
- 3.1': Length of the housing (compressed state)
- 3.2: Change in length of the housing
- 4: Drive shaft
- 4.1: Rotating direction of the drive shaft
- 5: Pliable Sheath
- 6: Downstream tubing
- 6.1: Downstream opening
- 8: Distal end region
- 9: Distal bearing
- 10: End part
- 10.1: Elongated portion of the polymer end part
- 10.2: Pigtail
- 11: Drive shaft cover
- 11.1: Proximal section
- 11.2: Central section
- 11.3: Distal section
- 11.4: Opening
- 11.5: Venting hole
- 12: Flexible tube
- 12': Flexible tube (outside configuration)
- 12": Flexible tube (inside configuration)
- 13: Heat conducting part
- 13': Inner side of the heat conducting part
- 13": Outer side of the heat conducting part
- 14: Spiral sleeve
- 14.1: Flat tape
- 14.2: Winding of the spiral sleeve
- 15: Cannula
- 16: Driving region
- 17: Motor
- 18.1: Heart
- 18.2: Aorta
- 18.3: Left ventricle
- 18.4: Aortic valve
- 19: Fluid Path

## Claims

1. A catheter device (1), comprising
- a drive shaft (4) extending from a driving region (16) of the catheter device (1) to a distal end region (8) of the catheter device (1),
- a rotor (2) which is attached to the drive shaft (4) in the distal end region (8) such that it can rotate together with the drive shaft,
- a distal bearing (9) for bearing a distal end of the drive shaft (4), wherein the distal bearing comprises a drive shaft cover (11), the drive shaft cover (11) being configured to cover a section of the drive shaft (4), which section extends distally of the rotor (2).

2. The catheter device (1) according to claim 1, wherein the distal bearing (9) comprises an end part (10), the drive shaft cover (11) being provided in a section of the drive shaft lying between the rotor (2) and the end part (10).

3. The catheter device (1) according to claim 2, wherein the drive shaft cover (11) extends along an entirety of the section of the drive shaft lying between the rotor (2) and the end part (10).

4. The catheter device (1) according to claim 2 or 3, wherein the drive shaft cover (11) extends into the end part (10).

5. The catheter device (1) according to any of the preceding claims,
wherein an inner diameter and/or an outer diameter of the drive shaft cover (11) varies along a length of the drive shaft cover (11), the drive shaft cover (11) in particular having two or three cylindrical sections with different outer diameters.

6. The catheter device (1) according to any of the preceding claims,
wherein an inner diameter of the drive shaft cover (11) at a proximal end of the drive shaft cover (11) is reduced with respect to an inner diameter of the drive shaft cover (11) at a distal end of the drive shaft cover (11), the inner diameter preferably being reduced by at least 0.02 mm and/or by at most 0.12 mm.

7. The catheter device (1) according to any of the preceding claims,
wherein the drive shaft cover (11) comprises a pliable section (11.2), the pliable section (11.2) preferably being arranged between a distal end of the rotor (2) and a proximal end of an end part (10) of the distal bearing (9).

8. The catheter device (1) according to claim 7, wherein the drive shaft cover (11) has a proximal section (11.1) having a smallest outer diameter, a central section having an increased outer diameter, the central section being the pliable section (11.2), and a distal section (11.3) having a largest outer diameter.

9. The catheter device (1) according to claim 7 or 8, wherein the pliable section (11.2) is provided by having at least one opening (11.4) in the drive shaft cover (11) in said pliable section (11.2).

10. The catheter device (1) according to claim 9, wherein the at least one opening (11.4) is a through-opening, connecting an inside of the drive shaft cover (11) to an outside of the drive shaft cover (11).

11. The catheter device (1) according to claim 10, wherein the at least one opening (11.4) comprises one or more slits, the one or more slits preferably having a course with a tangential component.

12. The catheter device (1) according to claim 11, wherein holes are provided at one end or at both ends of one or more of the slits, the holes having a diameter that is larger than a width of a given slit at which they are provided.

13. The catheter device (1) according to claim 11, wherein the slits have a closed course, completely surrounding the dive shaft cover (11) and thereby cutting the drive shaft cover into several segments, the segments preferably being connected to each other by having protrusions of one segment lying inside recesses of a neighboring segments.

14. The catheter device (1) according to any of claims 7-13, wherein the pliable section is designed as a hypotube.

15. The catheter device (1) according to any of claims 7-14, wherein the pliable section (11.2) is limp.
